(19) Europäisches Patentamt
European Patent Office
Office européen des brevets

(11) **EP 2 240 072 B1**

(12) **EUROPÄISCHE PATENTSCHRIFT**

(45) Veröffentlichungstag und Bekanntmachung des Hinweises auf die Patenterteilung:
**24.04.2013 Patentblatt 2013/17**

(21) Anmeldenummer: **08869322.1**

(22) Anmeldetag: **30.12.2008**

(51) Int Cl.:
*A61B 5/021* *(2006.01)*    *A61B 5/0285* *(2006.01)*
*A61B 5/053* *(2006.01)*

(86) Internationale Anmeldenummer:
**PCT/EP2008/011153**

(87) Internationale Veröffentlichungsnummer:
**WO 2009/086921 (16.07.2009 Gazette 2009/29)**

(54) **DRUCKMESSER, INSBESONDERE BLUTDRUCKMESSER, VERFAHREN ZUR BESTIMMUNG VON DRUCKWERTEN, VERFAHREN ZUM KALIBRIEREN EINES DRUCKMESSUNG UND COMPUTERPROGRAMM ZUR IMPLEMENTIERUNG DIESER VERFAHREN**

MANOMETER, PARTICULARLY BLOOD PRESSURE MANOMETER, METHOD FOR DETERMINING PRESSURE VALUES, METHOD FOR CALIBRATING A MANOMETER AND COMPUTER PROGRAM FRO IMPLEMENTING THESE METHODS

MANOMÈTRE, EN PARTICULIER SPHYGMOMANOMÈTRE, PROCÉDÉ DE DÉTERMINATION DE VALEURS DE PRESSION, PROCÉDÉ D'ÉTALONNAGE D'UN MANOMÈTRE ET PROGRAMME INFORMATIQUE POUR LA MISE EN OEUVRE DE CES PROCÉDÉS

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **11.01.2008 DE 102008003978**

(43) Veröffentlichungstag der Anmeldung:
**20.10.2010 Patentblatt 2010/42**

(73) Patentinhaber: **Fraunhofer-Gesellschaft zur Förderung der angewandten Forschung e.V.
80686 München (DE)**

(72) Erfinder:
• **DOUNIAMA, Christian
91054 Erlangen (DE)**
• **TOBOLA, Andreas
91334 Hemhofen (DE)**
• **WENTZLAFF, Holger
90411 Nürnberg (DE)**
• **BENZ, Michaela
91052 Erlangen (DE)**
• **NORGALL, Thomas
90542 Eckental (DE)**
• **COURONNE, Robert
91058 Erlangen (DE)**
• **WEIGAND, Christian
90556 Cadolzburg (DE)**

(74) Vertreter: **Burger, Markus
Schoppe Zimmermann
Stöckeler Zinkler & Partner
Postfach 246
82043 Pullach (DE)**

(56) Entgegenhaltungen:
EP-A- 1 157 658          EP-A2- 2 030 564
DE-A1- 10 061 189       US-A- 4 245 648
US-A1- 2004 162 493    US-A1- 2005 261 593

EP 2 240 072 B1

**Beschreibung**

Ausführungsbeispiele gemäß der Erfindung beziehen sich auf einen Druckmesser, auf einen Blutdruckmesser, auf ein Verfahren zum Bestimmen eines Druckwerts, auf ein Verfahren zum Kalibrieren eines Druckmessers und auf ein Computerprogramm.

Einige Ausführungsbeispiele gemäß der Erfindung beziehen sich auf Verfahren zur nichtinvasiven, kontinuierlichen Blutdruckmessung ohne Manschette mit Hilfe zweier Plethysmographen am Unterarm basierend auf einer Pulswellenlaufzeit und einer Pulswellenamplitude.

In vielen Situationen ist es wünschenswert, einen Blutdruck eines Menschen oder eines Tieres zu messen. Ganz allgemein ist im Übrigen die Messung eines Drucks, der beispielsweise in einer elastischen Fluidleitung (zum Beispiel einem Schlauch, einem dünnwandigen Rohr oder einem Blutgefäß) herrscht, erforderlich, um beispielsweise die Funktion einer Pumpe oder sonstige Prozessparameter zu überwachen.

Bei dem Vitalparameter "Blutdruck" handelt es sich beispielsweise um einen transmuralen Druck an arteriellen Gefäßwänden, der sich aus einer Differenz zwischen einem Innendruck und einem Außendruck des Gefäßes (bzw. Blutgefä-βes) ergibt. Durch eine Kontraktion des Herzens und einen gleichzeitigen Blutauswurf aus einem linken Ventrikel in der Aorta baut sich in einem Aortenbogen (beispielsweise innerhalb des Gefäßes) aufgrund der Inkompressibilität des Blutes ein Druck auf, der sich als Druckwelle (auch bekannt als Pulswelle) von der Aorta bis in eine Peripherie (eines Menschen oder eines Tieres) fortpflanzt und mit einer kurzzeitigen Volumenänderung (bzw. lokalen Volumenänderung bzw. Querschnittsänderung) eines betrachteten Gefäßabschnitts einhergeht. Man unterscheidet und definiert hierbei beispielsweise einen systolischen und einen diastolischen Blutdruckwert entsprechend als maximalen (systolischen) und minimalen (diastolischen) Gefäßdruck im Verlaufe eines Herzzyklus.

Es sind bereits verschiedene Verfahren zur Blutdruckmessung bekannt. Beispielsweise beschreibt die US 6,736,789 B1 ein Verfahren und eine Vorrichtung zur Blutbehandlung mit einer Vorrichtung zur kontinuierlichen Überwachung der außerhalb des Körpers erfolgenden Blutbehandlung. Ein Blutdruck eines Patienten oder eine Größe, die mit dem Blutdruck korreliert ist, wird gemessen und mit einem vorbestimmten Grenzwert verglichen. Wenn der gemessene Blutdruck oder dessen relative Veränderung unter den vorbestimmten Grenzwert fällt, wird ein Signal erzeugt, um die Behandlugsabfolge zu unterbrechen. Die kontinuierliche nicht-invasive Messung des Blutdrucks basiert auf einer Bestimmung der Ausbreitungsgeschwindigkeit oder Laufzeit der Pulswellen, die durch die Herz-Kontraktionen des Patienten und die Ausbreitung durch das Arteriensystem erzeugt werden. Zur Bestimmung der Puslwellengeschwindigkeit oder -laufzeit weist die Blutbehandlungsmaschine einen Elektro-Kardiograph sowie eine Einrichtung zum Detektieren der Pulswellen an einem Ort entfernt von dem Herzen des Menschen auf.

Die GB 2394178 A beschreibt ein Verfahren zum Kalibrieren eines Blutdrucküberwachungsapparats. Ein Blutdruckmessaparat wird kalibriert, indem der Blutdruck eines lebenden Körpers unter Verwendung eines Manometers gemessen wird. Ferner wird eine Referenzlaufzeit einer Blut-Pulswelle gemessen, die entlang eines Blutgefäßes vom Herz in eine Region, die von dem Herzen entfernt ist, wandert. Eine Ankunft der Blut-Pulswelle an der Fingerspitze wird unter Verwendung eines Plethysmographen detektiert. Eine erste Messung eines Blutdrucks und einer Laufzeit wird verwendet, um einen ersten Kalibrierungsdatenpunkt zu erzeugen. Die Region des Körpers, die von dem Herzen entfernt ist, wird dann ausgehend von einer Position auf gleicher Höhe mit dem Herzen angehoben und abgesenkt. Eine Laufzeit wird für die angehobene und abgesenkte Position gemessen. Eine Blutdruckdifferenz zwischen einem Zustand, in dem die Region angehoben oder abgesenkt ist, wird dann berechnet. Eine Laufzeit-Differenz zwischen der angehobenen und abgesenkten Position wird berechnet, und ein zweiter Datenpunkt wird aus der berechneten Blutdruckdifferenz und Laufzeitdifferenz erzeugt.

Die US 7,029,447 B2 beschreibt eine Vorrichtung und ein System zur nicht-invasiven Messung eines Blutdrucks eines Patienten. Das Verfahren umfasst folgende Schritte: Bestimmen eines mechanischen Herzschlag-Startzeitpunkts aus einem Impedanz-Kardiogramm-Signal, Detektieren einer Herzschlag-Pulsankunft-Zeit an einem peripheren Ort des Patienten, Berechnen einer Pulswellen-Laufzeit von dem Herzen zu dem peripheren Ort unter Verwendung des mechanischen Startzeitpunkts des Herzschlags und der Herzschlag-Puls-Ankunftszeit, sowie Berechnen eines Schätzwerts eines Blutdrucks des Patienten aus der Pulswellenlaufzeit.

Die US 6,648,828 B2 beschreibt eine kontinuierliche, nicht-invasive Methode zur Messung des Blutdrucks unter Verwendung einer Impedanz-Plethysmographie. Ein Blutdruck wird unter Verwendung einer Pulslaufzeit gemessen, die der Blut-Volumen-Puls benötigt, um sich zwischen zwei Orten in einem Tier auszubreiten. Eine Impedanz-Plethysmographie wird verwendet, um zu detektieren, wann der Blut-Volumen-Puls an einem Ort auftritt. Der Plethysmograph kann eine Brustkorb-Impedanz detektieren, um zu bestimmen, wann das Aorta-Herz-Ventil sich öffnet, oder er kann eine Impedanz an einem Ort einer Gliedmaße des Tieres detektieren. Das Auftreten des Blut-Volumen-Pulses an einem anderen Ort kann durch Impedanz-Plethysmographie oder eine andere Technik, wie z. B. Puls-Oximetrie, bestimmt werden. Eine Berechnung eines Herzschlag-Volumens kann verwendet werden, um eine Abweichung des Blutdrucks aufgrund von Effekten, die auf einer Dehnbarkeit des Blutgefäßes beruhen, zu kompensieren. Ein Blutdruckmonitor kann periodisch eine Referenzblutdruckmessung liefern, die verwendet wird, um die Ableitung des Blutdrucks basierend auf der Pulslaufzeit zu kalibrieren.

Die US 6,331,162 B1 beschreibt ein Gerät zur Messung einer Pulswellengeschwindigkeit. Das Gerät umfasst erste und zweite Plethysmographen-Sensoren, die mit einem Computer verbunden sind. Die Sensoren werden auf dem Rücken eines Patienten positioniert, um eine Puls-Wellenform-Information an zwei Orten entlang der Brust-Schlagader aufzuzeichnen. Zusätzlich wird ein Elektrokardiogram des Patienten aufgezeichnet. Sobald die Pulswellenformen und die Elektrokardiogram-Wellenform aufgezeichnet sind, werden verrauschte oder mit Artefakten behaftete Daten ausgeschlossen, und die Pulswellenformen werden unter Verwendung der Elektrokardiogrammdatenpunkte ermittelt. Danach werden die signalgemittelten Pulswellenformen analysiert, um einen Fußpunkt jeder Wellenform zu bestimmen und um eine Fußpunkt-zu-Fußpunkt-Laufzeit zwischen den zwei Sensoren zu bestimmen. Eine Pulswellengeschwindigkeit wird dann bestimmt, indem der Abstand zwischen den Sensoren durch die Fußpunkt-zu-Fußpunkt-Laufzeit geteilt wird.

Weitere Aspekte im Hinblick auf die Blutdruckmessung sind im Übrigen in der KR 000100697211 BA und in der KR 102006069032 AA beschrieben.

Die US 4,245,648 beschreibt ein Verfahren und eine Vorrichtung zur Messung eines Blutdrucks und einer Pulsrate. Das System umfasst einen Sensorkopf, der mit einer Arterie gekoppelt ist. Der Sensorkopf umfasst elektromechanische Wandler an zwei Orten, die jede periodische Arterien-Puls-Druck-Welle, die den ersten Ort bzw. den zweiten Ort passiert, in erste und zweite periodische elektrische Wellenformen umwandeln. Eine elektronische Schaltung analysiert die erste und die zweite periodische Wellenform, um die Anstiegszeit jeder periodischen Wellenform zu bestimmen. Die elektronische Schaltung analysiert auch die erste und die zweite periodische Wellenform, um die Laufzeit jeder Puls-Druck-Welle zwischen dem ersten und dem zweiten Ort zu bestimmen. Ein elektronischer Computer verwendet die Anstiegszeit und die Laufzeit sowie bestimmte Kalibrierungsdaten, um einen systolischen Blutdruck, einen diastolischen Blutdruck und eine Pulsrate zu bestimmen und anzuzeigen.

Die DE 100 61 189 A1 beschreibt ein Verfahren zur kontinuierlichen, nicht-invasiven Bestimmung eines arteriellen Blutdrucks durch Messung einer Pulswellenlaufzeit. Bei dem Verfahren werden an wenigstens zwei Körperbereichen eines Patienten Messsignale abgenommen. Aus einer Zeitdifferenz zwischen korrespondierenden Punkten der beiden Signale wird eine Pulswellenlaufzeit bestimmt, und daraus wird ein Wert für den Blutdruck abgeleitet. Um die Genauigkeit zu verbessern, ist vorgesehen, dass jeder Sensor mit wenigstens zwei Elektroden ein die Impedanz in dem Körperbereich repräsentierendes Messsignal aufnimmt. Aus den Messsignalen in den beiden Körperbereichen wird die Pulswellenlaufzeit bestimmt und daraus wird der mittlere arterielle Blutdruck bestimmt. Ferner wird auch der systolische und der diastolische Blutdruck bestimmt.

Die US 2005/0261593 A1 beschreibt Verfahren zur Messung eines Blutdrucks mit automatischen Kompensationen. Es wird ein Verfahren zur Messung eines arteriellen Blutdrucks beschrieben, das ein Detektieren eines Puls-Wellen-bezogenen Signals umfasst. Ferner wird ein Merkmal von den Signalen extrahiert und es wird ein Faktor bestimmt, der den arteriellen Blutdruck beeinflusst. Ferner wird der arterielle Blutdruck basierend auf dem Merkmal mit einer automatischen Kompensation der Faktoren bestimmt.

Die EP 1 157 658 A1 beschreibt einen Blutdruckmonitorapparat. Der Blutdruckmonitorapparat umfasst einen Apparat zum Erhalten einer Information, die sich auf eine Pulswellenausbreitungsgeschwindigkeit bezieht, mit der eine Pulswelle sich in einer Arterie ausbreitet. Ferner umfasst der Blutdruckmonitorapparat eine Vorrichtung zum Erhalten einer Information, die sich auf eine Pulsperiode bezieht. Zudem umfasst der Blutdruckmonitorapparat eine Vorrichtung zum Erhalten einer Information, die sich auf ein Blutvolumen bezieht, das in einem peripheren Körperteil fließt. Zudem umfasst der Blutdruckmonitorapparat eine Vorrichtung zur Beurteilung, ob der Blutdruck abnormal ist, wobei diese Vorrichtung feststellt, ob die oben genannten Informationen in zugehörigen Referenzbereichen liegen.

Die US 2004/0162493 A1 beschreibt eine Vorrichtung und ein Verfahren zur nichtinvasiven kontinuierlichen Bereitstellung von physiologischen Charakteristika. Messaufnehmer haben Strahlungssender und Detektoren und werden verwendet, um die Absorption von Blut in dem Gewebe des Patienten zu bestimmen, und um somit verschiedene Blut-Parameter zu bestimmen. Das Gerät hat zudem entweder einen Positionssensor zur Bestimmung der Position des Messaufnehmers in Bezug auf das Herz des Patienten oder einen Bewegungsgenerator zur Veränderung der Position des Messaufnehmers in Bezug auf das Herz des Menschen. Die genannte Schrift beschreibt auch Verfahren zur nichtinvasiven Beobachtung der physiologischen Charakteristika. Induzierte Positionsänderungen führen zu differenziellen hydrostatischen Drücken, die eine Messung von Blut-Parametern durch die Absorptionsfähigkeit erleichtern. In weiteren Ausführungsbeispielen werden Verzögerungen von Pulsankunftszeiten in gekoppelten Organen oder Gliedmaßen auf gegenüberliegenden Körperseiten gemessen, um eine Ausgabe des Herzens zu bestimmen.

Die EP 2 030 564 A2 beschreibt ein implantierbares Blutdruckmesssystem und entsprechende Verfahren. In der genannten Schrift werden implantierbare Systeme und Verfahren zu deren Verwendung beschrieben, um einen arteriellen Blutdruck auf einer chronischen Basis zu beobachten. Ein erstes Signal, das eine elektrische Aktivität des Patienten-Herzen beschreibt, und ein zweites Signal, das eine mechanische Aktivität des Patienten-Herzen beschreibt, werden unter Verwendung von implantierten Elektroden und eines implantierten Sensors erhalten. Durch Messung der Zeiten zwischen verschiedenen Merkmalen des ersten Signals relativ zu Merkmalen des zweiten Signals werden Werte erhalten, die den systolischen und den diastolischen Druck anzeigen. Derartige Merkmale werden beispielsweise verwendet, um eine Pulsspitzenankunftszeit zu bestimmen, welche dann verwendet wird, um einen Wert zu beschreiben, der den systolischen Druck anzeigt. Zusätzlich wird eine Spitze-zu-Spitze-Amplitude einer maximalen Spitze des zweiten Signals bestimmt. Der Wert, der den systolischen Druck anzeigt, wird dann verwendet, um den Wert, der den diastolischen Druck anzeigt, zu bestimmen.

In Anbetracht der obigen Ausführungen besteht ein Bedarf an einem Konzept zur Messung eines Drucks, das an die Eigenschaften elastischer Fluidleiter besonders gut angepasst ist.

Diese Aufgabe wird durch einen Druckmesser gemäß Anspruch 1, ein Verfahren zum Bestimmen von Druckwerten gemäß Anspruch 15, ein Verfahren zum Kalibrieren eines Druckmessers gemäß Anspruch 16 und ein Computerprogramm gemäß Anspruch 17 gelöst.

Einige Ausführungsbeispiele gemäß der Erfindung basieren auf der Erkenntnis, dass eine besonders zuverlässige Bestimmung eines Druckwertes basierend auf einem Messsignal erhalten werden kann, indem zunächst eine Laufzeitinformation, die eine Laufzeit einer Pulswelle zwischen einem ersten Ort und einem zweiten Ort beschreibt, ausgewertet wird, um einen ersten Blutdruckwert zu erhalten, und indem anschließend ein zweiter Druckwert unter Verwendung einer zweiten Abbildung, die einen von dem ersten Blutdruckwert abhängigen Zusammenhang zwischen einer Amplitudeninformation und einer Amplitude einer durch das Messsignal beschriebenen Pulswelle beschreibt, basierend auf der Amplitudeninformation bestimmt wird.

So wurde nämlich erkannt, dass ein Zusammenhang zwischen der Amplitudeninformation, die beispielsweise die Amplitude des Messsignals beschreibt, und der Amplitude einer durch das Messsignal beschriebenen Pulswelle aufgrund der Eigenschaften eines elastischen Fluidleiters von dem anliegenden Druck abhängig ist. Somit wird bei der Druckmessung beispielsweise die Tatsache berücksichtigt bzw. ausgenutzt, dass bei einem bereits stark gedehnten elastischen Fluidleiter (z. B. bei einem Blutgefäß) eine bestimmte Volumenänderung einer vergleichsweise großen Druckänderung entspricht, während hingegen bei einem weniger stark gedehnten Fluidleiter die gleiche Volumenänderung einer vergleichsweise kleineren Druckänderung entspricht.

Somit kann beispielsweise eine bestimmte Amplitude eines Messsignals, das beispielsweise ein (lokales) Volumen eines Fluidleiters bzw, eine Querschnittsfläche eines Fluidleiters (beispielsweise als Funktion der Zeit) beschreibt, abhängig von dem ersten Blutdruckwert auf eine Amplitude einer durch das Messsignal beschriebenen Pulswelle (also beispielsweise auf eine Differenz zwischen zwei Druckwerten, beispielsweise bei einer Blutdruckmessung auf eine Differenz zwischen einem systolischen und einem diastolischen Blutdruckwert) abgebildet werden.

Durch die indirekte Bestimmung des zweiten Druckwerts kann somit die Amplitudeninformation, die die Amplitude des Messsignals beschreibt, ausgewertet werden, wobei festzuhalten ist, dass sich eine Amplitude des Messsignals in der Regel sehr effizient und präzise bestimmen lässt. Um eine verbesserte Genauigkeit zu erzielen, wird allerdings bei der zweiten Abbildung, die einen Zusammenhang zwischen der Amplitude des Messsignals und einer Amplitude einer Pulswelle (bzw. einem Druckunterschied) beschreibt, der erste Druckwert mit berücksichtigt.

Durch die genannte Vorgehensweise kann beispielsweise ermöglicht werden, dass die Laufzeit nur für einen solchen ersten Druckwert ermittelt wird, für den eine Laufzeitmessung in besonders zuverlässiger bzw. präziser Weise möglich ist. Die Amplitude des Messsignals kann dann herangezogen werden, um den zweiten Druckwert, der beispielsweise durch eine Laufzeitmessung nur mit geringerer Präzision bestimmt werden könnte, basierend auf dem ersten Druckwert zu erhalten. Dies kann bei einigen Ausführungsbeispielen zu einer besonders genauen Messung führen, denn die Amplitude eines Messsignals ist in vielen Fällen ein in besonders präziser Weise ermittelbarer Parameter eines Messsignals.

Somit ist es gemäß einigen Ausführungsbeispielen nicht erforderlich, den zweiten Druckwert (z. B. einen minimalen bzw. kleinere Druckwert einer Pulswelle) unmittelbar durch eine Laufzeitmessung zu bestimmen, was unter Umständen schwierig bzw. fehlerbehaftet sein kann. Vielmehr kann beispielsweise der untere bzw. kleinere Druckwert (z. B. ein diastolischer Druckwert) indirekt, basierend auf der Bestimmung eines oberen bzw. größeren Druckwerts (z. B. eines systolischen Druckwerts) und basierend auf einer Amplitude eines Messsignals ermittelt werden. Die Amplitude des Messsignals kann dabei, abhängig von dem bestimmten oberen bzw. höheren Druckwert (systolischen Druckwert) auf den niedrigeren bzw. unteren Druckwert abgebildet werden, wozu die von dem ersten Druckwert abhängige zweite Abbildung eingesetzt werden kann.

Zusammenfassend ist somit festzuhalten, dass gemäß einigen Ausführungsbeispielen ein indirektes Konzept zur Druckbestimmung bzw. zur Ermittlung eines Druckwerts geschaffen wird, das bei der Bestimmung mancher Druckwerte (z. B. bei der Bestimmung eines unteren bzw. niedrigeren Druckwerts oder eines diastolischen Druckwerts) zu einer besonders hohen Präzision führt.

Einige Ausführungsbeispiele gemäß der Erfindung beziehen sich im Übrigen auf einen Blutdruckmesser, bei dem das hierin beschriebene Konzept zur Messung eines Blutdruckwertes z. B. eines systolischen Blutdruckwerts oder eines diastolischen Blutdruckwerts, eingesetzt wird.

Bei einigen Ausführungsbeispielen kann das erfindungsgemäße Konzept auch in effizienter Weise eingesetzt werden, um zwei verschiedene Blutdruckwerte, beispielsweise sowohl einen systolischen Blutdruckwert als auch einen diastolischen Blutdruckwert, zu bestimmen.

Einige Ausführungsbeispiele gemäß der Erfindung beziehen sich im Übrigen auf ein Verfahren zur Messung von Druckwerten.

Weitere Ausführungsbeispiele der Erfindung beziehen sich auf ein Verfahren zum Kalibrieren eines Druckmessers.

Einige Ausführungsbeispiele gemäß der Erfindung basieren auf dem Kerngedanken, dass bei einer Kalibrierung eines Druckmessers eine Bestimmung von zwei Abbildungen zu besonders guten Ergebnissen führt. Eine erste Abbildung, die bei der Kalibrierung bzw. bei dem Verfahren zum Kalibrieren eines Druckmessers erstellt bzw. bestimmt wird, oder für die bei dem Kalibrieren Parameter bestimmt werden, beschreibt einen Zusammenhang zwischen Laufzeiten der Pulswellen und zugehörigen Druckwerten. Eine zweite Abbildung wird daraufhin basierend auf der ersten Abbildung erstellt. Indem die zweite Abbildung basierend auf der ersten Abbildung erstellt wird, kann erreicht werden, dass ein qualitativer Verlauf der zweiten Abbildung einen druckabhängigen Zusammenhang zwischen lokalen Volumenwerten oder lokalen Volumenänderungswerten des Fluidleiters, in dem sich die Pulswelle ausbreitet, und zugehörigen Druckwerten oder Druckunterschieden beschreibt. Die Bestimmung der zweiten Abbildung wird also dadurch erleichtert, dass die zweite Abbildung auf der ersten Abbildung basiert, da nämlich ein enger Zusammenhang zwischen einer Abbildung, die einen Zusammenhang zwischen Laufzeiten der Pulswellen und zugehörigen Druckwerten beschreibt, und einer Abbildung, die einen Zusammenhang zwischen lokalen Volumenwerten und zugehörigen Druckwerten beschreibt, besteht. Somit kann, indem die zweite Abbildung basierend auf der ersten Abbildung erhalten wird, zumindest ein qualitativer Verlauf der zweiten Abbildung ohne hohen messtechnischen Aufwand festgelegt werden. Vielmehr ergibt sich bei einigen Ausführungsbeispielen die zweite Abbildung bzw. deren qualitativer Verlauf unmittelbar, ohne Heranziehung weiterer Messwerte, aus der ersten Abbildung.

Eine Skalierungsgröße der zweiten Abbildung kann im Übrigen basierend auf zwei Referenzdruckwerten und zumindest zwei entsprechenden Signalwerten eines Messsignals in sehr einfacher Weise festgelegt werden.

Somit ist festzuhalten, dass sowohl die erste Abbildung als auch die zweite Abbildung in besonders effizienter Weise bei einer Kalibrierung eines Druckmessers bestimmt werden können. Zur Festlegung der ersten Abbildung und der zweiten Abbildung ist nur eine sehr geringe Anzahl von Messungen bzw. Messwerten erforderlich.

Insofern schaffen einige Ausführungsbeispiele gemäß der Erfindung eine besonders effiziente Kalibrierung, wobei die durch die Kalibrierung erhaltenen Abbildungen bzw. Abbildungsvorschriften wiederum, wie schon oben erläutert, zur Bestimmung besonders präziser Ergebnisse ausgenutzt werden können.

Ausführungsbeispiele gemäß der Erfindung werden nachfolgend Bezug nehmend auf die beiliegenden Figuren näher erläutert. Es zeigen:

Fig. 1    ein Blockschaltbild eines Druckmessers, gemäß einem Ausführungsbeispiel der Erfindung;

Fig. 2a    eine schematische Darstellung einer Pulswelle in einem elastischen Fluidleiter zu einem ersten Zeitpunkt;

Fig. 2b    eine schematische Darstellung der Pulswelle in dem elastischen Fluidleiter zu einem zweiten Zeitpunkt;

Fig. 3    eine schematische Darstellung eines Arms, an dem zwei Plethysmographen angeordnet sind;

Fig. 4    eine graphische Darstellung von zwei Plethysmographensignalen von zwei an verschiedenen Orten angeordneten Plethysmographen;

Fig. 5    eine schematische Darstellung einer Vorgehensweise zur Bestimmung von Druckwerten basierend auf einer Laufzeitinformation und einer Amplitudeninformation, gemäß einem Ausführungsbeispiel der Erfindung;

Fig. 6a    eine schematische Darstellung einer Vorgehensweise bei einer Bestimmung eines zweiten Druckwerts basierend auf einem ersten Druckwert und einer Amplitudeninformation, gemäß einem Ausführungsbeispiel der Erfindung;

Fig. 6b    eine schematische Darstellung einer Vorgehensweise bei einer Bestimmung eines zweiten Druckwertes und eines dritten Druckwertes basierend auf einem ersten Druckwert und einer Amplitudeninformation, gemäß einem Ausführungsbeispiel der Erfindung;

Fig. 7    ein Blockschaltbild eines Druckmessers, gemäß einem weiteren Ausführungsbeispiel der Erfindung;

Fig. 8a    einen ersten Teil einer schematischen Darstellung einer Vorgehensweise zur Kalibrierung eines Druckmessers, gemäß einem Ausführungsbeispiel der Erfindung;

Fig. 8b    einen zweiten Teil einer schematischen Darstellung einer Vorgehensweise zur Kalibrierung eines Druckmessers, gemäß einem Ausführungsbeispiel der Erfindung;

Fig. 8c    eine schematische Darstellung von verschiedenen Pulswellen, die zur Kalibrierung eines Druckmessers herangezogen werden können;

Fig. 9    eine zusammengefasste schematische Darstellung einer Vorgehensweise bei einer Kalibrierung eines Druckmessers und bei einer Berechnung eines Druckwerts;

Fig. 10    ein Flussdiagramm eines Verfahrens zum Bestimmen eines ersten Druckwertes und eines zweiten Druckwertes basierend auf einer Laufzeitinformation und einer Amplitudeninformation; und

Fig. 11    ein Flussdiagramm eines Verfahrens zur Kalibrierung eines Druckmessers, gemäß einem Ausführungsbeispiel der Erfindung.

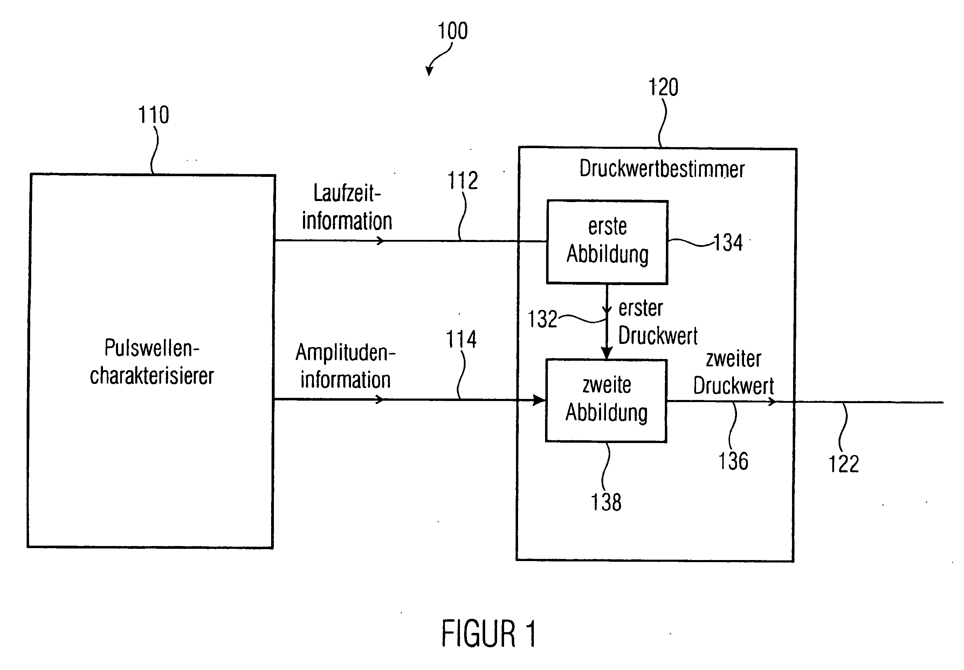

Fig. 1 zeigt ein Blockschaltbild eines Druckmessers, gemäß einem Ausführungsbeispiel der Erfindung. Der Druckmesser gemäß der Fig. 1 ist in seiner Gesamtheit mit 100 bezeichnet. Der Druckmesser 100 umfasst einen Pulswellencharakterisierer 110. Der Pulswellencharakterisierer 110 ist ausgelegt, um eine Laufzeitinformation 112 zu liefern, die eine Laufzeit einer Pulswelle zwischen einem ersten Ort und einem zweiten Ort beschreibt. Der Pulswellencharakterisierer 110 ist ferner ausgelegt, um eine Amplitudeninformation 114 zu liefern, die eine Veränderung eines auf der Pulswelle basierenden Messsignals zwischen zwei Phasen der Pulswelle beschreibt. Der Druckmesser umfasst ferner einen Druckwertbestimmer 120. Der Druckwertbestimmer 120 ist ausgelegt, um die Laufzeitinformation 112 und die Amplitudeninformation 114 zu empfangen, und um basierend darauf zumindest einen Druckwert 122 (hier als "zweiter Druckwert" bezeichnet) zu liefern.

Der Druckwertbestimmer 120 ist beispielsweise ausgelegt, um einen ersten Druckwert 132, der einen Druck eines Fluides in einer ersten Phase eines pulsierenden Flusses beschreibt, basierend auf der Laufzeitinformation 112 und unter Verwendung einer ersten Abbildung 134, die die Laufzeit einer Pulswelle auf den ersten Druckwert abbildet, zu erhalten.

Der Druckwertbestimmer 120 ist ferner ausgelegt, um einen zweiten Druckwert 136, der beispielsweise dem von dem Druckwertbestimmer 120 als Ausgabeinformation gelieferten Druckwert 122 entspricht, und der beispielsweise einen Druck des Fluides in einer zweiten Phase des pulsierenden Flusses beschreibt, basierend auf dem ersten Druckwert 132 und der Amplitudeninformation 114 zu bestimmen. Dabei wird beispielsweise eine zweite Abbildung 138 verwendet, die einen von dem ersten Druckwert 132 abhängigen Zusammenhang zwischen dem ersten Druckwert, dem zweiten Druckwert 136 und der Amplitudeninformation 114 beschreibt.

Im Hinblick auf die Funktionalität des Druckmessers 100 ist somit festzuhalten, dass der zweite Druckwert 136 in einem mehrstufigen System bzw. Verfahren unter Verwendung mehrerer Abbildungen 134, 138 basierend auf der Laufzeitinformation 112 und der Amplitudeninformation 114 erzeugt wird. Die Laufzeitinformation 112 wird zunächst im Rahmen der ersten Abbildung 134 ausgewertet, um den ersten Druckwert 132 zu bestimmen. Anschließend wird der zweite Druckwert 136, ausgehend von dem ersten Druckwert, unter Verwendung der zweiten Abbildung 138 und unter Berücksichtigung der Amplitudeninformation 114 ermittelt. Die zweite Abbildung 138 kann beispielsweise in direkter oder indirekter Form beschreiben, wie stark sich der zweite Druckwert 136 von dem ersten Druckwert 132 unterscheidet. Dabei kann die zweite Abbildung beispielsweise beschreiben, dass ein Unterschied zwischen dem ersten Druckwert 132 und dem zweiten Druckwert 136 nicht nur von der Amplitudeninformation 114, sondern auch von dem ersten Druckwert 132 abhängig ist. Somit kann beispielsweise die nicht-linear mit dem Druck veränderliche Ausdehnung eines elastischen Fluidleiters (z. B. eines Blutgefäßes) bei der Berechnung des zweiten Druckwertes 136 berücksichtigt werden, wobei beispielsweise der erste Druckwert 132 eine Information darüber liefern kann, wie stark der elastische Fluidleiter in einem Zustand, in dem die Laufzeitinformation 112 gewonnen wird, gedehnt ist.

Somit kann der Druckmesser 100 beispielsweise eine besonders präzise bzw. zuverlässige Information über den zweiten Druckwert 136 liefern, wobei sowohl die Laufzeitinformation als auch die Amplitudeninformation ausgenutzt werden.

Weitere Details werden im Folgenden beschrieben.

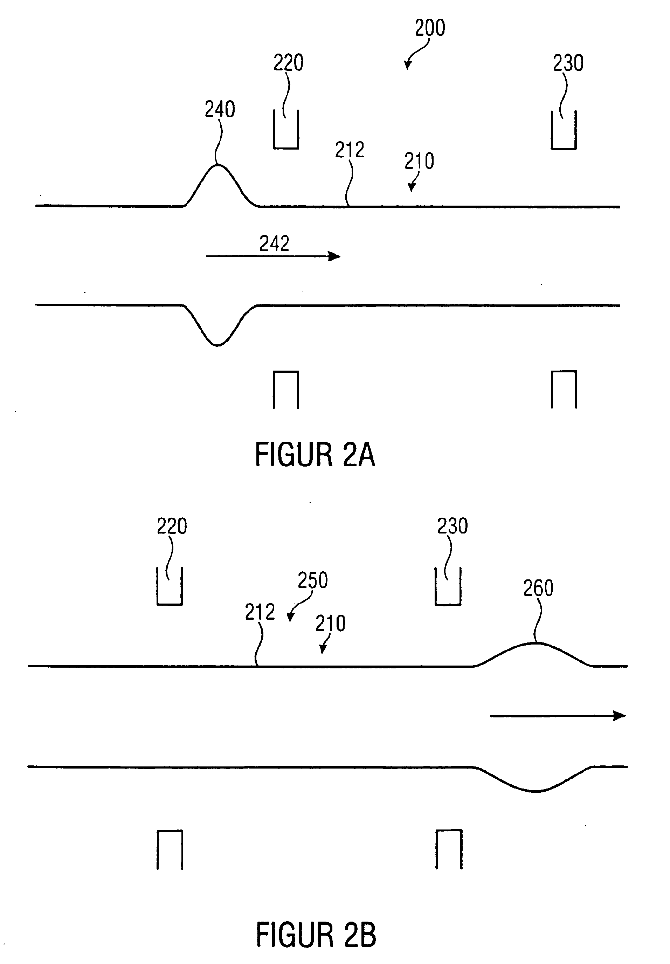

Zum besseren Verständnis zeigen die Fig. 2a und 2b schematische Darstellungen eines elastischen Fluidleiters, in dem sich eine Pulswelle mit der Zeit ausbreitet.

Die schematische Darstellung der Fig. 2a ist in ihrer Gesamtheit mit 200 bezeichnet. Die schematische Darstellung der Fig. 2b ist in ihrer Gesamtheit mit 250 bezeichnet. Im Übrigen sind in den Figuren 2a und 2b gleiche Bezugszeichen verwendet, um gleiche Einrichtungen bzw. Merkmale zu bezeichnen.

Die schematische Darstellung 200 zeigt einen Querschnitt durch einen Fluidleiter 210. Eine elastische Wand des Fluidleiters ist mit 212 bezeichnet. Eine Ausbreitung einer Pulswelle in dem Fluidleiter 210 ist beispielsweise daran erkennbar, dass die Wand 212 des Fluidleiters 210 an einem Ort, an dem sich die Pulswelle gerade befindet, nach außen gewölbt ist.

Die schematische Darstellung 200 gemäß Fig. 2a zeigt beispielsweise, in schematischer Weise, einen ersten Sensor 220 sowie einen zweiten Sensor 230. Der erste Sensor ist beispielsweise ausgelegt, um eine lokale Vergrößerung (bzw. Veränderung) eines Querschnitts des Fluidleiters 210 an einem ersten Ort zu erkennen. Der zweite Sensor 230 ist beispielsweise ausgelegt, um eine lokale Vergrößerung (bzw. Veränderung) eines Querschnitts des Fluidleiters 210 an einem zweiten Ort zu erkennen.

Der erste Sensor 220 liefert somit beispielsweise ein erstes Mess-Signal, das beispielsweise eine Funktion eines lokalen Durchmessers, einer lokalen Querschnittsfläche oder eines lokalen Volumens (beispielsweise an einem vorgegebenen ersten Ort oder für einen ersten Abschnitt) des elastischen Fluidleiters 210 an einem ersten Ort bzw. für einen ersten Abschnitt des Fluidleiters beschreibt. Der zweite Sensor 230 liefert entsprechend beispielsweise ein zweites Mess-Signal, das einen lokalen Durchmesser, eine lokale Querschnittsfläche oder ein lokales Volumen (beispielsweise eines Abschnitts) des Fluidleiters 210 (beispielsweise an einem vorgegebenen zweiten Ort bzw. für einen zweiten Abschnitt) beschreibt.

In den Figuren 2a und 2b ist ferner eine Pulswelle erkennbar. Beispielsweise zeigt die schematische Darstellung 200 der Fig. 2a eine durch die Pulswelle bedingte QuerschnittsVergrößerung des elastischen Fluidleiters 210. Die Pulswelle breitet sich beispielsweise von links nach rechts aus, wie dies durch einen Pfeil 242 dargestellt ist. Somit breitet sich die Pulswelle zwischen einem ersten Zeitpunkt, zu dem der in der schematischen Darstellung 200 gezeigte Zustand vorliegt, und einem zweiten Zeitpunkt, zu dem der in der schematischen Darstellung 250 gezeigte Zustand vorliegt, in der durch den Pfeil 242 bezeichneten Richtung aus. Somit ergibt sich zu dem zweiten Zeitpunkt eine lokale Querschnittsvergrößerung 260, die aus der schematischen Darstellung 250 ersichtlich ist. Mit der Ausbreitung der Pulswelle bewegt sich somit die lokale Vergrößerung der Querschnittsfläche des elastischen Fluidleiters 210 an den beiden Sensoren 220, 230 vorbei. Die entsprechende Bewegung der lokalen Durchmesservergrößerung bzw. lokalen Querschnittsflächenvergrößerung bzw. lokalen Volumenvergrößerung spiegelt sich im Übrigen in entsprechenden Impulsen in dem ersten Mess-Signal und in dem zweiten Mess-Signal wieder, wie im Folgenden noch beschrieben wird.

Im Übrigen kann sich eine Form der Pulswelle bzw. eine Form der durch die Pulswelle verursachten Durchmesservergrößerung bzw. Querschnittsflächenvergrößerung im Laufe der Ausbreitung der Pulswelle verändern. Beispielsweise kann im Laufe der Ausbreitung aus einer räumlich eng konzentrierten Pulswelle eine räumlich ausgebreitete Pulswelle werden, wie dies aus den Fig. 2a und 2b ersichtlich ist (vgl. lokale Querschnittsvergrößerungen 240 und 260). Dementsprechend kann beispielsweise das durch den ersten Sensor 220 gelieferte Mess-Signal einen kurzen und intensiven impulsförmigen Verlauf aufweisen, während hingegen das durch den zweiten Sensor 230 gelieferte Mess-Signal einen längeren und dafür weniger intensiven Verlauf aufweist.

Im Übrigen sei darauf hingewiesen, dass die anhand von Fig. 2a und 2b erläuterte Ausbreitung einer Pulswelle in gleicher Weise in einem Blutgefäß oder in einem anderen elastischen Fluidleiter auftreten kann. Diesbezüglich bestehen auch im Hinblick auf die Mess-Signale, die sich bei einer Blutdruckmessung ergeben im Vergleich zu den Mess-Signalen, die sich bei einer Meldung eines Drucks in einer technischen Anlage oder in einem technischen System ergeben, keine gravierenden Unterschiede. Somit kann das beschriebene Konzept sowohl für eine Blutdruckmessung als auch für eine Druckmessung in technischen Anlagen oder Systemen eingesetzt werden.

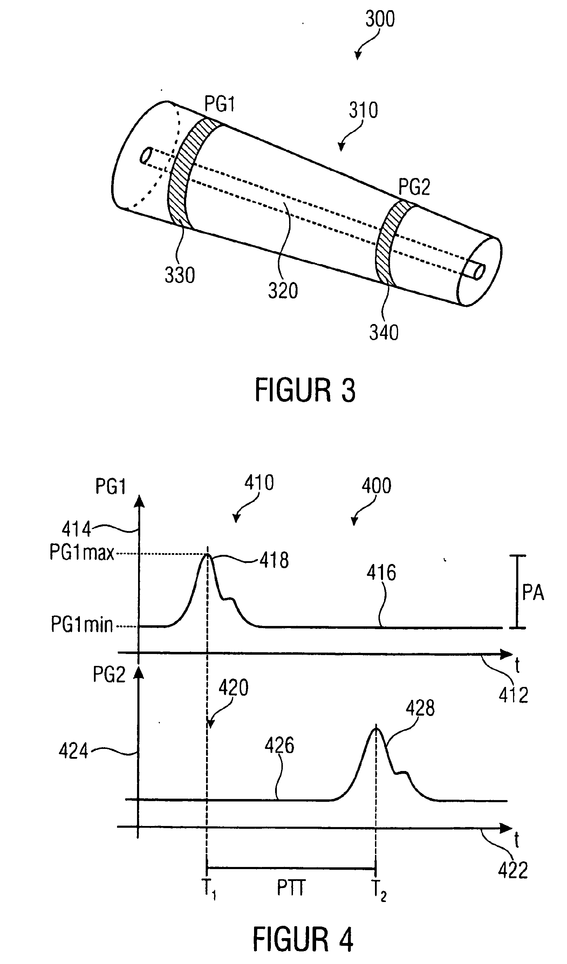

Fig. 3 zeigt eine schematische Darstellung eines Unterarms 310 und eines Verlaufs einer Ellenarterie (Arteria ulnaris) und einer Speichenarterie (Arteria radialis). Die Arterien sind beispielsweise durch eine gestrichelte Linie 320 angedeutet, wobei hier nicht ausdrücklich zwischen der Ellenarterie und der Speichenarterie unterschieden wird.

Die schematische Darstellung gemäß der Fig. 3 ist im Übrigen in einer Gesamtheit mit 300 bezeichnet und zeigt ferner, schematisch, einen ersten Plethysmographen 330, der an einem ersten Ort bzw. an einer ersten Position an dem Unterarm 310 angeordnet ist. Der erste Plethysmograph 330 ist beispielsweise ausgelegt, um lokale Veränderungen eines Durchmessers, einer Querschnittfläche oder eines (lokalen) Volumens der Ellenarterie und/oder der Speichenarterie zu erfassen und durch ein erstes Mess-Signal zu beschreiben. Die schematische Darstellung 300 zeigt ferner einen zweiten Plethysmographen 340, der an einer zweiten Position bzw. an einem zweiten Ort des Unterarms 310 angeordnet ist. Der zweite Plethysmograph 340 ist beispielsweise ausgelegt, um lokale Veränderungen eines Durchmessers, einer Querschnittsfläche oder eines (lokalen) Volumens der Ellenarterie und/oder der Speichenarterie an einem zweiten Ort bzw. an einer zweiten Position zu erfassen und basierend darauf ein zweites Mess-Signal zu erzeugen. Die Plethysmographen 330, 340 sind in der Fig. 3 schematisch als Bänder bzw. als bandförmige Vorrichtungen gezeigt, die um den Unterarm 310 angeordnet sind. Allerdings können auch andersartige Plethysmographen verwendet werden, beispielsweise solche, die eine Volumenänderung einer oder mehrerer Arterien auf optischem Wege (beispielsweise durch Durchleuchtung des Unterarms), durch eine Impedanzmessung an dem Unterarm oder durch eine Kapazitätsmessung an dem Unterarm bestimmen, um das erste Mess-Signal und das zweite Mess-Signal zu erhalten. Im Übrigen können auch andere Vorrichtungen als Plethysmographen verwendet werden, um das erste Mess-Signal und das zweite Mess-Signal so zu erhalten, dass die Mess-Signale eine Pulswelle in der Ellenarterie und/oder in der Speichenarterie an verschiedenen Orten entlang des Unterarms beschreiben.

Im Übrigen sei darauf hingewiesen, dass anstelle des Unterarms 310 beispielsweise auch ein Oberarm, ein Unterschenkel oder ein Oberschenkel verwendet werden können. Im Übrigen kann die Blutdruckmessung natürlich auch an anderen Körperteilen eines Menschen oder eines Tiers erfolgen.

Fig. 4 zeigt eine schematische Darstellung von zeitlichen Signalverläufen, beispielsweise des ersten Plethysmographen 330 (PG 1) und des zweiten Plethysmographen 340 (PG 2). Die zeitlichen Signalverläufe können beispielsweise in der gezeigten Weise als Basis zur Berechnung einer Laufzeit bzw. Pulslaufzeit (auch als PTT bezeichnet) und einer Pulsamplitude (auch als PA bezeichnet) verwendet werden. Die schematische Darstellung der Fig. 4 ist in ihrer Gesamtheit mit 400 bezeichnet. Die schematische Darstellung 400 umfasst eine erste Signaldarstellung 410, die beispielsweise das von dem ersten Plethysmographen 330 gelieferte Mess-Signal (auch kurz als PG 1 bezeichnet) beschreibt. Eine Abszisse 412 beschreibt beispielsweise eine Zeit, und eine Ordinate 414 beschreibt beispielsweise in willkürlichen Einheiten eine Größe des durch den ersten Plethysmographen 330 gelieferten ersten Mess-Signals. Eine Kurve 416 beschreibt einen zeitlichen Verlauf des ersten Mess-Signals.

Die graphische Darstellung 400 umfasst eine zweite Signaldarstellung 420. Die zweite Signaldarstellung 420 umfasst eine Abszisse 422, an der die Zeit t angetragen ist, sowie eine Ordinate 424, die eine Größe des von dem zweiten Plethysmographen 340 gelieferten zweiten Mess-Signals in willkürlichen Einheiten beschreibt. Eine Kurve 426 beschreibt eine zeitliche Entwicklung des zweiten Mess-Signals.

In der Fig. 4 sind beispielsweise die Abszissen bzw. Zeitachsen 412, 422 aufeinander ausgerichtet.

Eine Amplitude des ersten Mess-Signals kann beispielsweise als eine Differenz zwischen einem Maximalwert PG1max und einem Minimalwert PG1min des ersten Mess-Signals innerhalb eines bestimmten Zeitabschnitts definiert sein, wie dies beispielsweise in der ersten Signaldarstellung 410 definiert ist.

Aus den Signaldarstellungen 410, 420 ist ersichtlich, dass beispielsweise ein Maximum des ersten Mess-Signals, das durch die Kurve 416 beschrieben ist, zu einem ersten Zeitpunkt T1 auftritt, während hingegen ein Maximum des zweiten Mess-Signals, das durch die Kurve 426 beschrieben ist, zu einem zweiten Zeitpunkt T2 auftritt. Diesbezüglich sei darauf hingewiesen, dass beispielsweise das Maximum 418 des ersten Mess-Signals und das Maximum des 428 des zweiten Mess-Signals einander zugeordnet sind bzw. zusammengehören, also beispielsweise durch die Ausbreitung einer einzigen Pulswelle bedingt sind. Ein Zeitversatz zwischen dem Maximum 418 des ersten Mess-Signals und dem Maximum 428 des zweiten Mess-Signals wird hier als Laufzeit PTT bezeichnet.

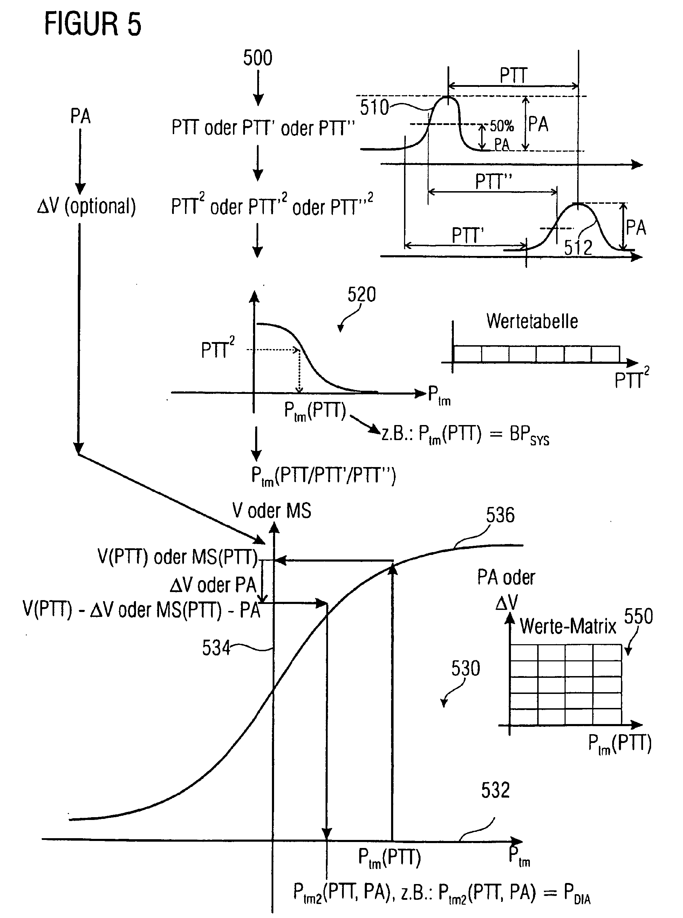

Im Folgenden wird anhand der Fig. 5 erläutert, wie der hierin beschriebene Druckmesser bzw. Blutdruckmesser, z.B. der Druckmesser 100 gemäß Fig. 1, basierend auf einer Laufzeitinformation (beispielsweise basierend auf der Laufzeitinformation 112, auch mit PTT oder PTT' oder PTT" bezeichnet) und der Amplitudeninformation (beispielsweise der Amplitudeninformation 114, auch mit PA bezeichnet) einen Druckwert ermitteln kann.

Die schematische Darstellung gemäß der Figur 5 ist in ihrer Gesamtheit mit 500 bezeichnet und beschreibt beispielsweise ein Verfahren, wie es durch den Druckwertbestimmer 120 ausgeführt werden kann. In anderen Worten, der Druckwertbestimmter 120 kann beispielsweise ausgelegt sein, um das anhand der Fig. 5 beschriebene Verfahren auszuführen.

Im Folgenden soll zunächst erläutert werden, wie die Laufzeitinformation 112 erhalten werden kann. Die Laufzeitinformation 112 kann beispielsweise durch Auswertung des ersten Mess-Signals (das in der hierin erläuterten Art und Weise erzeugt werden kann) und des zweiten Mess-Signals (das ebenso in der hierin erläuterten Weise erzeugt werden kann) bestimmt werden. Beispielsweise können zur Bestimmung der Laufzeitinformation 112 zusammengehörige Pulse 510, 512 des ersten Mess-Signals (Puls 510) und des zweiten Mess-Signals (Puls 512) ausgewertet werden, um die Laufzeitinformation 112 zu erhalten. Unter zusammengehörigen Pulsen werden Pulse verstanden, die durch die gleiche Pulswelle verursacht werden, und die beispielsweise dadurch erkannt werden können, dass sie innerhalb eines bestimmten zeitlichen Abstands liegen.

Bei einem Ausführungsbeispiel kann die Laufzeitinformation 112 beispielsweise beschreiben, wie viel Zeit zwischen einem Maximum des ersten Pulses 510 (in dem ersten Mess-Signal) und einem Maximum des zweiten Pulses 512 (in dem zweiten Mess-Signal) vergeht, wie dies in der Fig. 5 gezeigt ist.

Bei einem weiteren Ausführungsbeispiel kann die Laufzeitinformation 112 beschreiben, welche Zeit zwischen einem Beginn des ersten Pulses 510 und einem Beginn des zweiten Pulses 512 vergeht. Ein Beginn des ersten Pulses 510 kann beispielsweise als Zeitpunkt definiert sein, zu dem das Mess-Signal gegenüber einem Ruhe-Signalwert um einen bestimmten Betrag angestiegen ist, wobei der entsprechende bestimmte Betrag beispielsweise als ein absoluter Wert oder als ein Prozentsatz der Signalamplitude PA definiert sein kann. Beispielsweise kann der Beginn des ersten Pulses 510 als Zeitpunkt definiert sein, zu dem das erste Mess-Signal ausgehend von einem Ruhe-Wert um 5% oder 10% der Signalamplitude PA ansteigt. Ein Beginn bzw. Anfang des zweiten Pulses 512 kann in entsprechender Weise definiert sein. Die entsprechende Laufzeitinformation PTT', die die Zeit beschreibt, die zwischen dem Anfang des ersten Pulses 510 und dem Anfang des zweiten Pulses 512 vergeht, kann beispielsweise eine Zeitdauer beschreiben, die größer oder kleiner als eine Teildauer zwischen Maxima der Pulse 510, 512 ist, da sich ja Formen der Pulse 510, 512 unterscheiden können.

Bei einem weiteren Ausführungsbeispiel kann beispielsweise die Laufzeitinformation 112 eine Zeitdauer zwischen einer Mitte einer steigenden Flanke des ersten Pulses 510 und einer Mitte einer steigenden Flanke des zweiten Pulses 512 beschreiben. Unter der Mitte der steigenden Flanke wird beispielsweise ein Zeitpunkt verstanden, zu dem ein Puls ausgehend von einem Ruhe-Wert um 50% der zugehörigen Signalamplitude PA angestiegen ist. Die entsprechende Definition ist in der Fig. 5 veranschaulicht. Der resultierende Wert der Laufzeitinformation 112 ist im Übrigen mit PTT" bezeichnet.

Im Übrigen sei darauf hingewiesen, dass die Laufzeitinformationswerte PTT, PTT' und PTT" alternativ verwendet werden können. In anderen Worten, es ist ausreichend, wenn die Laufzeitinformation 112 einen der genannten Werte PTT, PTT', PTT" beschreibt.

Gemäß einem Ausführungsbeispiel kann der durch die Laufzeitinformation beschriebene Laufzeitwert in einem ersten Schritt quadriert werden, um einen ersten quadrierten Laufzeitwert $PTT^2$ bzw. $PTT'^2$ oder $PTT"^2$ zu erhalten. In einem zweiten Schritt kann beispielsweise basierend auf dem quadrierten Laufzeitwert unter Verwendung einer ersten Abbildung 520 ein zugehöriger erster Blutdruckwert $P_{tm}(PTT)$ bestimmt werden. Die erste Abbildung 520 kann somit einen Zusammenhang zwischen dem quadrierten Laufzeitwert $PTT^2$ und einem zugehörigen transmuralen Druck $P_{tm}(PTT)$ beschreiben. Die Schreibweise "(...)" bedeutet hier "als Funktion von ..." oder "in Abhängigkeit von ..." oder "zu ... gehörig".

Die erste Abbildung 520 kann beispielsweise durch einen geschlossenen Ausdruck, der beispielsweise eine Gauß-Funktion beschreibt, oder durch Parameter eines geschlossenen Ausdrucks beschrieben sein. Die erste Abbildung 520 kann alternativ beispielsweise auch durch eine geeignete Wertetabelle beschrieben sein. Andere Möglichkeiten zur Beschreibung einer ersten Abbildung sind natürlich auch einsetzbar. Die erste Abbildung 520 kann im Übrigen durch eine analoge Schaltung oder durch eine digitale Schaltung (z.B. eine digitale Rechenschaltung) realisiert werden. Diesbezüglich sind also die verschiedensten Möglichkeiten verwendbar.

Im Übrigen sei darauf hingewiesen, dass das in Fig. 5 beschriebene Konzept bei einem Blutdruckmesser eingesetzt werden kann. Beschreibt beispielsweise die Laufzeitinformation bzw. der entsprechende Laufzeitwert die Zeitdauer PTT zwischen den Maxima der Pulse 510, 512, so kann man beispielsweise durch die erste Abbildung 520 basierend auf dem quadrierten Laufzeitwert als einen ersten Druckwert bzw. Blutdruckwert einen systolischen Blutdruckwert, einen diastolischen Blutdruckwert oder einen mittleren Blutdruckwert, erhalten. Wird als Laufzeitwert der Laufzeitwert PTT' verwendet, so kann die erste Abbildung 520 beispielsweise als ersten Druckwert einen systolischen Blutdruckwert, einen diastolischen Blutdruckwert oder einen mittleren Blutdruckwert liefern. Wird als Laufzeitwert der Laufzeitwert PTT" verwendet, so liefert die erste Abbildung 520 als den ersten Druckwert beispielsweise, zumindest näherungsweise, einen systolischen Blutdruckwert, einen mittleren Blutdruckwert oder einen diastolischen Blutdruckwert.

Ganz allgemein lässt sich festhalten, dass durch die Anwendung der ersten Abbildung 520 auf den verwendeten Laufzeitwert (PTT oder PTT' oder PTT") ein entsprechender Druckwert $P_{tm}(PTT)$ bzw. $P_{tm}(PTT')$ bzw. $P_{tm}(PTT")$ als erster Druckwert erhalten wird.

In einem dritten Schritt können dann die Amplitudeninformation 114 bzw. ein entsprechender Amplitudenwert (PA) sowie der erste Druckwert $P_{tm}(PTT)$ oder $P_{tm}(PTT')$ oder $P_{tm}(PTT")$ verwendet werden, um einen zweiten Druckwert zu erhalten.

Anhand der Fig. 5 wird im Folgenden beschrieben, wie das Verfahren durchgeführt werden kann, wenn beispielsweise die Laufzeitinformation 112 den Laufzeitwert PTT beschreibt (also die Zeitdauer zwischen Maxima zweier Pulse 510, 512).

Eine zweite Abbildung 530 kann beispielsweise einen Zusammenhang zwischen einem Druck $P_{tm}$ und einem lokalen Durchmesser, einer lokalen Querschnittsfläche oder einem lokalen Volumen V beschreiben. Alternativ dazu kann die zweite Abbildung 530 auch einen Zusammenhang zwischen dem Druck $P_{tm}$ (in einem flexiblen Fluidleiter) und einem zugehörigen Mess-Signal MS (beispielsweise eines entsprechenden Sensors oder Plethysmographen) beschreiben. Bei einigen Ausführungsbeispielen kann im Übrigen das Mess-Signal MS des Sensors zumindest näherungsweise proportional zu dem Volumen V des elastischen Fluidleiters sein, so dass eine Abbildung, die einen Zusammenhang zwischen dem Druck $P_{tm}$ und dem lokalen Volumen V beschreibt, qualitativ zumindest näherungsweise identisch zu einer Abbildung, die einen Zusammenhang zwischen dem Druck $P_{tm}$ und dem Mess-Signal MS beschreibt, ist. Somit beschreibt die zweite Abbildung 530 insgesamt - auch dann, wenn die zweite Abbildung primär einen Zusammenhang zwischen Druck und Volumen beschreibt - einen Zusammenhang zwischen zwei Druckwerten und einer Amplitude des Messsignals.

Bei einigen Ausführungsbeispielen kann eine Amplitude PA eines Messsignals zumindest näherungsweise proportional zu einer Volumenänderung ΔV sein.

Bei einigen Ausführungsbeispielen kann die zweite Abbildung unmittelbar eine Zuordnung zwischen einer Amplitude PA eines Messsignals und einer Druckdifferenz (zum Beispiel zwischen dem ersten Druckwert und dem zweiten Druckwert) beschreiben.

Bei einigen Ausführungsbeispielen kann die zweite Abbildung eine Zuordnung zwischen einer Volumenveränderung ΔV und einer Druckdifferenz (zum Beispiel zwischen dem ersten Druckwert und dem zweiten Druckwert) beschreiben. Zusätzlich kann die zweite Abbildung in diesem Falle als Skalierung einen Zusammenhang (zum Beispiel eine Proportionalität) zwischen einer Amplitude PA des Messsignals und der Volumenänderung $\Delta V$ beschreiben (zum Beispiel durch einen entsprechenden Proportionalitätskonstante "Faktor").

Bei einigen Ausführungsbeispielen kann die zweite Abbildung eine Zuordnung zwischen dem ersten Druckwert und dem zweiten Druckwert in Abhängigkeit von einer Amplitude PA eines Messsignals beschreiben. Der Unterschied zwischen dem ersten Druckwert und dem zweiten Druckwert ist dabei beispielsweise nicht nur von der Amplitude PA abhängig, sondern auch von dem ersten Druckwert.

Bei einigen Ausführungsbeispielen kann die zweite Abbildung eine Zuordnung zwischen dem ersten Druckwert und dem zweiten Druckwert in Abhängigkeit von einer Volumenänderung $\Delta V$ eines Messsignals beschreiben. Der Unterschied zwischen dem ersten Druckwert und dem zweiten Druckwert ist dabei beispielsweise nicht nur von der Amplitude PA abhängig, sondern auch von dem ersten Druckwert. Zusätzlich kann die zweite Abbildung in diesem Falle als Skalierung einen Zusammenhang (zum Beispiel eine Proportionalität) zwischen einer Amplitude PA des Messsignals und der Volumenänderung $\Delta V$ beschreiben (zum Beispiel durch einen entsprechenden Proportionalitätskonstante "Faktor").

Aus den obigen Ausführungen ist ersichtlich, dass eine Vielzahl von Möglichkeiten besteht, um einen Zusammenhang zwischen dem ersten Druckwert, dem zweiten Druckwert und der Amplitudeninformation in Form einer Abbildung zu beschreiben. Alle oben beschriebenen Möglichkeiten und auch weitere hier nicht beschriebene Möglichkeiten können in der hierin beschriebenen Vorrichtung und in dem hierin beschriebenen Verfahren verwendet werden.

Eine mögliche Abbildung, die als "zweite Abbildung" verwendet werden kann, ist in der Fig. 5 bei Bezugszeichen 530 beschrieben. Beispielsweise ist zur Veranschaulichung an einer Abszisse 532 ein Druck $P_{tm}$ angetragen. An einer Ordinate 534 ist beispielsweise eine Größe eines lokalen Volumens V oder eine Größe eines Mess-Signals MS angetragen. Eine Kurve 536 beschreibt beispielsweise einen Zusammenhang zwischen dem Druck $P_{tm}$ und einem Wert des lokalen Volumens V oder einen Zusammenhang zwischen dem Druck $P_{tm}$ und einem Wert des Mess-Signals MS.

Bei dem anhand der Fig. 5 beschriebenen Ausführungsbeispiel kann beispielsweise der in einem zweiten Schritt ermittelte Druck $P_{tm}$ (hier auch als $P_{tm}(PTT)$ bezeichnet) unter Verwendung der zweiten Abbildung 530 auf einen Volumenwert $V(PTT)$ oder auf einen Mess-Signalwert $MS(PTT)$ abgebildet werden. Ist beispielsweise der Amplitudenwert PA bekannt, so kann folglich ein modifizierter Mess-Signalwert $MS(PTT)-PA$ bestimmt werden. Alternativ kann der Amplitudenwert PA auf einen Volumen-Änderungswert $\Delta V$ abgebildet werden. Somit kann ein modifizierter Volumenwert $V(PTT)-\Delta V$ bestimmt werden. Basierend auf dem entsprechend erhaltenen Wert $MS(PTT)-PA$ bzw. $V(PTT)-\Delta V$ kann somit unter Verwendung der zweiten Abbildung der Druckwert $P_{tm2}$ (PTT, PA) bestimmt werden.

Die zweite Abbildung 530 kann dabei beispielsweise verwendet werden, um zu dem Druckwert $P_{tm}(PTT)$ den zugehörigen Volumenwert V oder Mess-Signalwert MS zu erhalten und um zu dem modifizierten Volumenwert $V(PTT)-\Delta V$ oder modifizierten Mess-Signalwert $MS(PTT)-PA$ den zweiten Druckwert $P_{tm2}$ zu erhalten, wie dies in der Fig. 5 dargestellt ist.

Ein Zusammenhang bzw. eine Differenz zwischen $P_{tm}(PTT)$ und $P_{tm2}(PTT, PA)$ ist gemäß der zweiten Abbildung 530 nicht nur von der Mess-Signal-Amplitude PA oder der Volumenänderung $\Delta V$ abhängig, sondern auch von dem ersten Druckwert $P_{tm}(PTT)$. Diese Abhängigkeit ergibt sich aus der NichtLinearität der Kurve 530, welche wiederum die Eigenschaften eines elastischen Fluidleiters, wie beispielsweise eines Blutgefäßes, wiederspiegelt.

Ferner sei darauf hingewiesen, dass es sich bei dem anhand der Fig. 5 beschriebenen Ausführungsbeispiel bei dem zweiten Druckwert beispielsweise um einen diastolischen Druckwert $P_{DIA}$ handeln kann.

Im Übrigen sei darauf hingewiesen, dass die zweite Abbildung 530 in verschiedenen Formen repräsentiert werden kann. Beispielsweise kann die zweite Abbildung durch eine parametrisierte geschlossene Funktionsvorschrift beschrieben werden.

Alternativ kann die zweite Abbildung durch eine Wertetabelle bzw. Zuordnungstabelle beschrieben werden, die eine Zuordnung zwischen Druckwerten und Volumenwerten oder zwischen Druckwerten und Amplitudenwerten beschreibt.

Alternativ kann die zweite Abbildung durch eine Werte-Matrix repräsentiert werden, die beispielsweise verschiedenen Kombinationen aus dem ersten Druckwert $P_{tm}(PTT)$ und der Mess-Signal-Amplitude PA unterschiedliche Werte für den zweiten Druckwert $P_{tm2}$ zuordnet. Die Werte-Matrix, die beispielhaft in der Fig. 5 bei Bezugszeichen 550 gezeigt ist, kann beispielsweise beschreiben, dass ein Unterschied zwischen dem ersten Druckwert und dem zweiten Druckwert nicht nur von der Mess-Signal-Amplitude PA bzw. der Volumenänderung $\Delta V$ abhängig ist, sondern auch von dem ersten Druckwert $P_{tm}(PTT)$ selbst.

Während Bezug nehmend auf Fig. 5 beispielsweise eine Beschreibung der zweiten Abbildung 530 durch eine Kurve 536, durch eine Wertetabelle oder durch eine Werte-Matrix 550 beschrieben wurde, kann die zweite Abbildung 530 natürlich auch in anderer Weise realisiert werden. Beispielsweise kann eine analoge Schaltung entworfen werden, die die zweite Abbildung 530 realisiert. Im Übrigen sind auch andere Realisierungsformen möglich, beispielsweise durch Auswerten eines in geschlossener Form beschriebenen Funktionsverlauf, durch Einsatz mehrerer Wertetabellen oder durch ähnliche Maßnahmen.

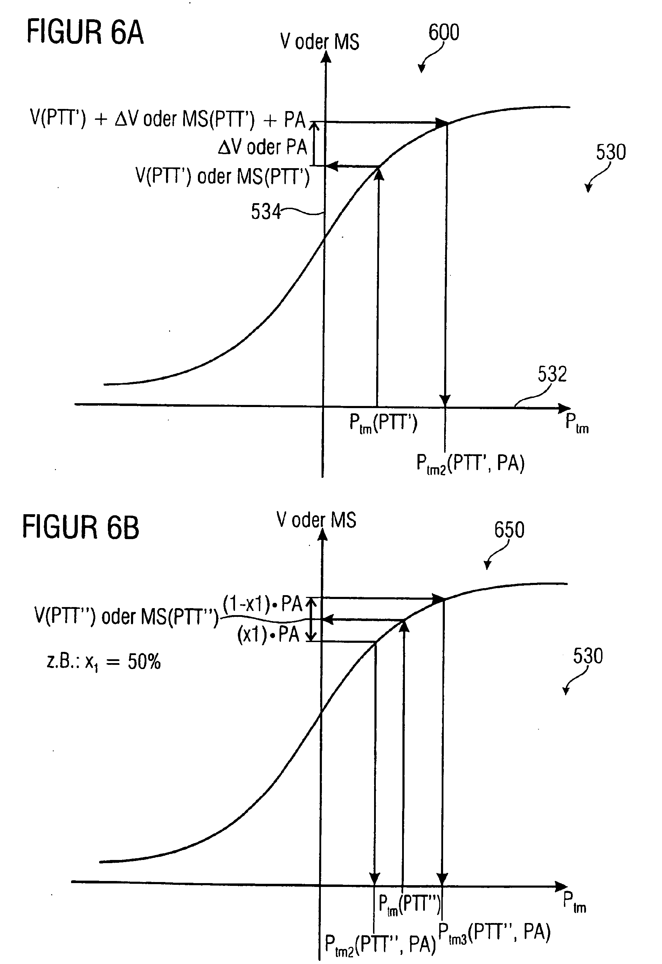

Im Folgenden wird anhand der Figuren 6a und 6b kurz beschrieben, wie anhand des ersten Druckwertes der zweite Druckwert bei einigen alternativen Ausführungsbeispielen ermittelt werden kann. Mit anderen Worten, Fig. 6a zeigt eine schematische Darstellung, die eine Bestimmung des zweiten Druckwertes basierend auf dem ersten Druckwert unter Verwendung der Mess-Signal-Amplitude beschreibt. Die graphische Darstellung gemäß der Fig. 6a ist in ihrer Gesamtheit mit 600 bezeichnet.

Die in Fig. 6a beschriebenen Vorgehensweise kann im Übrigen den dritten Schritt, wie er anhand der Fig. 5 beschrieben wurde, ersetzen, beispielsweise wenn als erster Druckwert zumindest näherungsweise ein diastolischer Druckwert erhalten wird. Es werde hier beispielsweise angenommen, dass der Wert $P_{tm}(PTT')$ einen diastolischen Druckwert bezeichnet. Allerdings können bei einigen Ausführungsbeispielen auch $P_{tm}(PTT)$ oder $P_{tm}(PTT'')$ einen entsprechenden diastolischen Druckwert beschreiben. Im Übrigen sei darauf hingewiesen, dass gleiche Einrichtungen bzw. Merkmale bzw. Schritte in den Figuren 5, 6a und 6b mit gleichen Bezugszeichen bezeichnet sind.

Wie aus der Fig. 6a unschwer ersichtlich ist, kann beispielsweise zu dem ersten, diastolischen Druckwert (zum Bespiel $P_{tm}(PTT')$) unter Verwendung der zweiten Abbildung 530 ein zugehöriger Volumenwert oder Mess-Signal-Wert (beispielsweise $V(PTT')$ oder $MS(PTT')$) ermittelt werden. Entsprechend können ein modifizierter Volumenwert (beispielsweise $V(PTT')+\Delta V$) oder ein modifizierter Mess-Signal-Wert (beispielsweise $MS(PTT')+PA$) ermittelt werden, woraus wiederum, beispielsweise unter erneuter Auswertung der zweiten Abbildung, der zweite Druckwert (beispielsweise $P_{tm2}(PTT', PA)$) ermittelt werden kann. Die entsprechende Vorgehensweise ist aus Fig. 6a ohne Weiteres ersichtlich.

Fig. 6b zeigt eine schematische Darstellung, die eine Bestimmung des zweiten Druckwertes und eines dritten Druckwertes basierend auf dem ersten Druckwert unter Verwendung der Mess-Signal-Amplitude beschreibt. Die graphische Darstellung gemäß der Fig. 6b ist in ihrer Gesamtheit mit 650 bezeichnet. Die anhand der Fig. 6b beschriebene Vorgehensweise kann im Übrigen den dritten Schritt, wie er beispielsweise anhand der Fig. 5 beschrieben wurde, ersetzen, beispielsweise wenn als erster Druckwert ein mittlerer Druckwert erhalten wird. Es werde hier beispielsweise angenommen, dass der Wert $P_{tm}(PTT'')$ einen mittleren Druckwert bezeichnet. Allerdings können bei einigen Ausführungsbeispielen auch $P_{tm}(PTT)$ oder $P_{tm}(PTT')$ einen entsprechenden mittleren Druckwert beschreiben.

Wie aus der Fig. 6b unschwer ersichtlich ist, kann beispielsweise zu dem ersten Druckwert $P_{tm}(PTT'')$ (oder $P_{tm}(PTT)$ oder $P_{tm}(PTT')$) unter Verwendung der zweiten Abbildung 530 ein zugehöriger Volumenwert (beispielsweise $V(PTT'')$) oder Mess-Signal-Wert (beispielsweise $MS(PTT'')$) ermittelt werden. Entsprechend kann beispielsweise ein oberer Volumenwert (beispielsweise $V(PTT'')+(1-x_1)\cdot\Delta V$) oder ein oberer Mess-Signal-Wert (beispielsweise $MS(PTT'')+(1-x_1)\cdot PA$) ermittelt

werden. Aus dem oberen Volumenwert oder dem oberen Mess-Signal-Wert kann beispielsweise unter erneuter Anwendung der zweiten Abbildung 530 der dritte Druckwert (beispielsweise $P_{tm3}(PTT'', PA)$) bestimmt werden. Ferner kann beispielsweise ein unterer Volumenwert (beispielsweise $V(PTT'')-x_1\cdot\Delta V$) oder ein unterer Mess-Signal-Wert (beispielsweise $MS(PTT'')-x_1\cdot PA$) berechnet werden. Aus dem unteren Volumenwert oder dem unteren Mess-Signal-Wert kann dann, beispielsweise unter erneuter Verwendung der zweiten Abbildung 530, der zweite Druckwert (beispielsweise $P_{tm2}(PTT'', PA)$) bestimmt werden. Die Vorgehensweise ist im Übrigen aus der Figur 6b ohne Weiteres ersichtlich.

Unter Verwendung der anhand von Fig. 6b beschriebenen Vorgehensweise können beispielsweise ausgehend von einer Messung einer Impulslaufzeit PTT, PTT' oder PTT'', die bei manchen Ausführungsbeispielen einen mittleren Druckwert repräsentiert, und basierend auf einer Messung der Pulsamplitude PA sowohl der zweite Druckwert als auch der dritte Druckwert ermittelt werden. Bei einem Ausführungsbeispiel ist die Messung des Laufzeitwertes PTT'' besonders zuverlässig bzw. präzise, da die Pulse 510, 512 in einem Bereich in der Nähe Ihres amplitudenmäßigen Mittelwertes eine besonders große Steigung aufweisen. Der Wert $x_1$ kann dabei angeben, dass einer der Pulse 510, 512 zu einem Zeitpunkt, zu dem der Laufzeitwert PTT'' gemessen wird, um $x_1\cdot PA$ über einem Ruhewert liegt. Unter Verwendung des anhand der Fig. 6b beschriebenen Verfahrens können somit beispielsweise ein systolischer und ein diastolischer Druckwert ermittelt werden, wobei es nicht erforderlich ist, einen Laufzeitwert PTT zwischen den Maxima der Pulse 510, 512 zu bestimmen. Vielmehr ist es ausreichend, den Laufzeitwert PTT'' zu bestimmen.

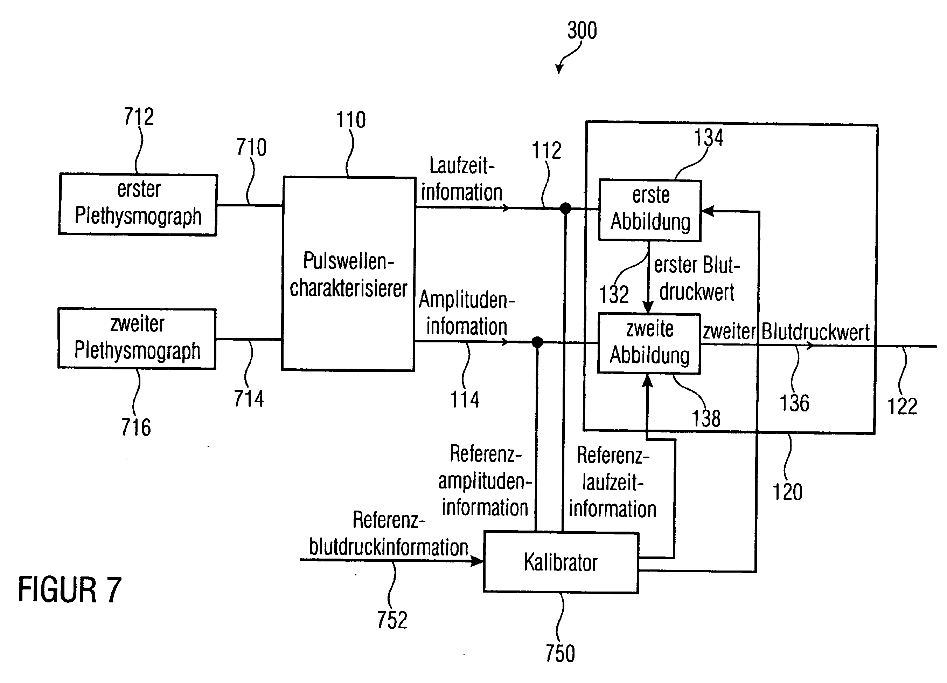

Fig. 7 zeigt ein Blockschaltbild eines Druckmessers gemäß einem Ausführungsbeispiel der Erfindung. Der Druckmesser gemäß der Fig. 7 ist in seiner Gesamtheit mit 700 bezeichnet. Im Übrigen kann es sich bei dem Druckmesser 700 beispielsweise um einen Blutdruckmesser handeln, aber auch um einen Druckmesser zum Einsatz in der industriellen oder sonstigen Druckmessung.

Der Druckmesser 700 gemäß der Fig. 7 ist dem Druckmesser 100 der Fig. 1 sehr ähnlich, so dass gleiche Einrichtungen, Merkmale oder Signale in den Figuren 1 und 7 mit gleichen Bezugszeichen bezeichnet sind.

Der Druckmesser 700 kann im Übrigen, wie auch der Druckmesser 100, beispielsweise ausgelegt sein, um ein erstes Mess-Signal 710 von einem ersten Plethysmographen 712 und ein zweites Mess-Signal 714 von einem zweiten Plethysmographen 716 zu empfangen. Das erste Mess-Signal 710 und das zweite Mess-Signal 714 können beispielsweise dem Pulswellen-Charakterisierer 110 zugeführt werden, wobei der Pulswellen-Charakterisierer 110 ausgelegt sein kann, um die Laufzeitinformation 112 und die Amplitudeninformation 114 basierend auf den Mess-Signalen 710, 714 zu bestimmen. Die Plethysmographen 712, 716 können im Übrigen bei einigen Ausführungsbeispielen Teil des Druckmessers sein. Bei anderen Ausführungsbeispielen können die Plethysmographen 712, 716 auch getrennt von dem Druckmesser sein und beispielsweise von einem anderen Hersteller hergestellt oder geliefert werden. Im Übrigen können die Plethysmographen 712, 716 durch andere Mess-Einrichtungen ersetzt werden, die geeignet sind, um Mess-Signale 710, 714 zu liefern, die eine Ausbreitung beispielsweise einer Pulswelle beschreiben. Im Übrigen kann beispielsweise auch eines der Mess-Signale 710, 714 in einer medizinischen Anwendung durch einen Elektrokardiographen geliefert werden, oder durch eine Einrichtung zur Messung einer Brustkorb-Impedanz.

Bei dem Druckmesser 700 ist im Übrigen ein Kalibrator 750 vorgesehen, der ausgelegt ist, um die erste Abbildung 134 und die zweite Abbildung 138 geeignet einzustellen, um eine Kalibrierung des Druckmessers 700 zu erreichen. Mit anderen Worten, die erste Abbildung 134 und die zweite Abbildung 138 sind bei dem vorliegenden Ausführungsbeispiel nicht fest vorgegeben, sondern einstellbar, beispielsweise durch Festlegung von Parametern einer geschlossenen Funktionsbeschreibung (z.B. von Parametern a, b einer Gauß-Funktion der Form $PTT^2 = a \cdot \exp(-b \cdot P_{tm}^2)$). Alternativ kann die erste Abbildung 134 aber auch durch eine veränderbare Wertetabelle, die beispielsweise in einem Speicher abgelegt ist, dargestellt sein. Die Einträge der Wertetabelle können beispielsweise unmittelbar im Rahmen einer Kalibrierung (z.B. durch Eintragen von Paaren von gemessenen Werten) erhalten werden. Die Einträge der Wertetabelle, die die erste Abbildung beschreibt, können alternativ dazu beispielsweise durch Skalierung einer vorgegebenen Funktion (beispielsweise der oben angegebenen Gauß-Funktion, die mit Parametern a und b parametrisiert ist) erhalten werden. Die vorgegebene Funktion kann beispielsweise durch eine geschlossene Funktionsvorschrift beschrieben sein oder in einer fest vorgegebenen Wertetabelle gespeichert sein.

Die zweite Abbildung 138 kann beispielsweise einstellbar sein. Die zweite Abbildung 138 kann beispielsweise durch eine parametrisierte geschlossene Funktionsvorschrift oder durch eine Wertetabelle oder Wertematrix beschrieben werden. Im Übrigen existieren verschiedene Beschreibungsformen der zweiten Abbildung 138, wie dies oben bereits erläutert wurde.

Der Kalibrator 750 ist ausgelegt, um bei einer Kalibrierung die erste Abbildung 134 und die zweite Abbildung 138 geeignet einzustellen. Zu diesem Zweck empfängt der Kalibrator 750 bei einer Kalibrierung beispielsweise zusammengehörige Werte der Laufzeitinformation 112, der Amplitudeninformation 114 und einer Referenz-Druckinformation bzw. Referenz-Blutdruckinformation 752. Die Referenz-Blutdruckinformation 752 beschreibt z.B. einen Blutdruckwert, der durch ein weiteres Druck-Messgerät oder Blutdruck-Meßgerät unter denselben äußeren Bedingungen (z.B. in zeitlich nahem Zusammenhang) gemessen wurde, zu denen auch die zugehörigen Werte der Laufzeitinformation 112 und der Amplitudeninformation 114 bestimmt wurden.

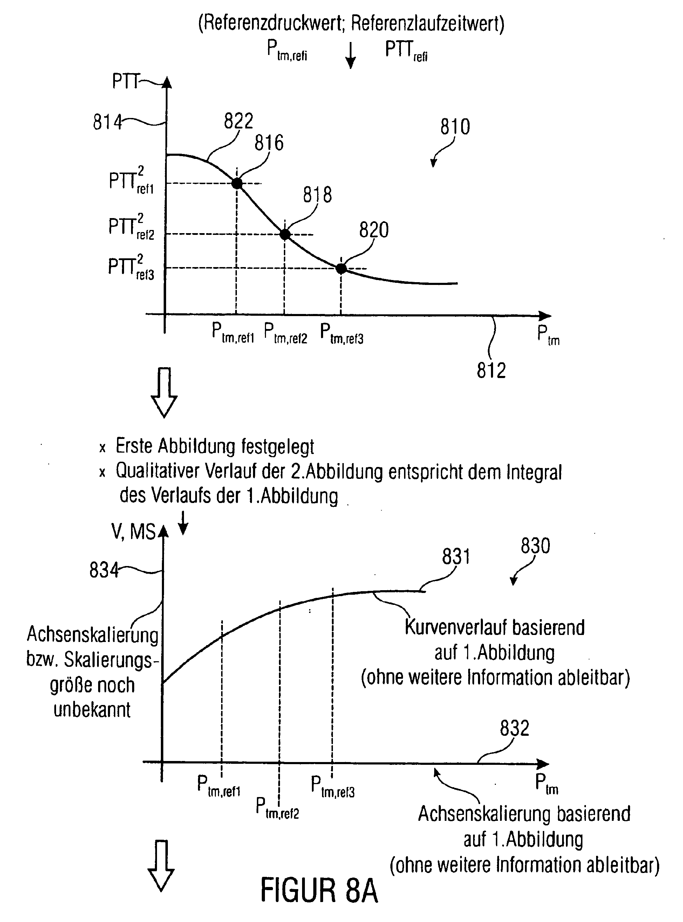

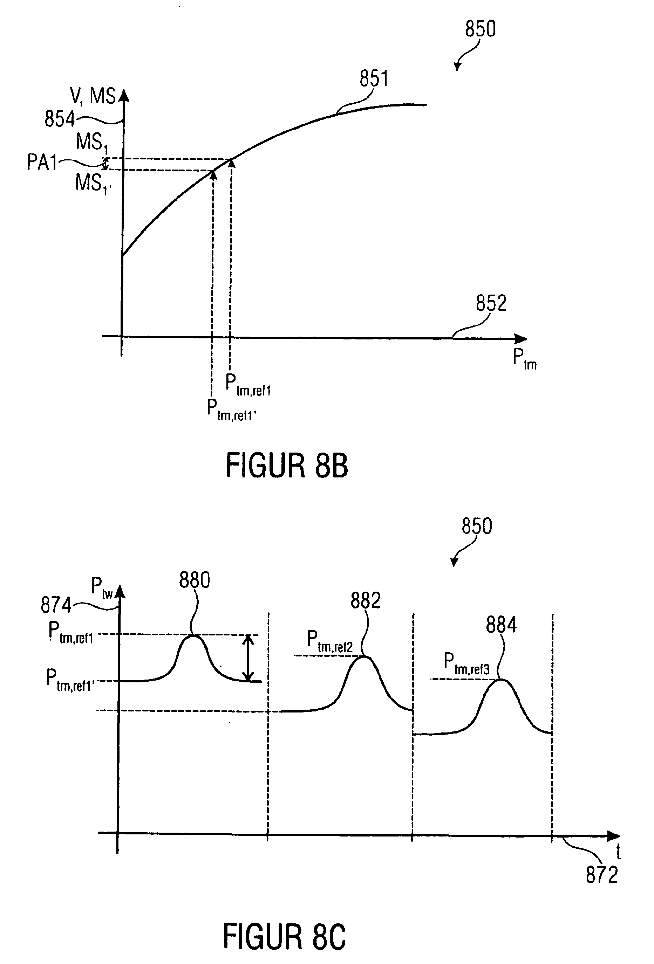

Details im Hinblick auf eine Vorgehensweise bei der Kalibrierung werden im Folgenden anhand der Figuren 8a und 8b beschrieben. In anderen Worten, die Figuren 8a und 8b zeigen eine schematische Darstellung der Vorgehensweise bei der Kalibrierung.

Eine schematische Darstellung 810 zeigt, wie aufgrund von mehreren Paaren von gemessenen Werten der Referenz-Druckinformation 752 (hier auch als Referenzdruck-Werte bezeichnet) und der Laufzeitinformation 112 (hier auch als Referenz-Laufzeitwerte bezeichnet) die erste Abbildung bestimmt bzw. festgelegt werden kann. Bei einem Ausführungsbeispiel ist ein qualitativer Verlauf der ersten Abbildung beispielsweise durch eine vorgegebene geschlossene Funktionsvorschrift gegeben, die einen oder mehrere veränderbare Parameter aufweist (z.B. durch eine Funktionsvorschrift, die eine Gauß-Kurve beschreibt, wobei Parameter a, b noch unbestimmt sind). Alternativ kann ein qualitativer Verlauf der ersten Abbildung auch durch eine vorgegebene Wertetabelle beschrieben sein, die durch eine entsprechende Skalierung an die gemessenen Referenz-Druckwerte bzw. Referenz-Laufzeitwerte angepasst wird.

Die erste schematische Darstellung 810 veranschaulicht die Bestimmung der ersten Abbildung anhand einer Darstellung der Abbildung als eine Kurve. An einer Abszisse 812 ist beispielsweise ein Druck $P_{tm}$ angetragen, und an einer Ordinate 814 ist beispielsweise ein Quadrat eines Laufzeitwertes $(PTT^2)$ angetragen. Ein qualitativer Verlauf der Abbildung 810 kann beispielsweise durch einen Gauß-artigen Zusammenhang zwischen dem Druckwert $P_{tm}$ und einem zugehörigen quadrierten Laufzeitwert $PTT^2$ festgelegt sein, wobei zumindest näherungsweise gelten kann: $PTT^2 = a \cdot \exp(-b \cdot P_{tm}^2)$. Die Abbildung 810 zeigt Paare von zusammengehörigen MessWerten, die als Punkte 816, 818, 820 dargestellt sind. Die Punkte 816, 818, 820 repräsentieren also Paare aus einem gemessenen Referenz-Druckwert und einem zugehörigen gemessenen Referenz-Laufzeitwert. Die Parameter des genannten Gauß-artigen Zusammenhangs, der durch eine Linie bzw. Kurve 822 dargestellt ist, werden bei der Kalibrierung so eingestellt, dass der Gauß-artige Verlauf möglichst wenig von den Referenz-Messwerte-Paaren abweicht. In anderen Worten, die Parameter a, b des Gauß-förmigen Verlaufs werden so gewählt, dass die Linie 822 möglichst nahe bei den Punkten 816, 818, 820 verläuft. Dazu kann beispielsweise ein Verfahren zur Minimierung von Fehlerquadranten eingesetzt werden, wie dies wohl bekannt ist. Im Übrigen sei darauf hingewiesen, dass bereits zwei Paare von Referenz-Messwerten ausreichend sind, um zwei Parameter des Gauß-artigen Zusammenhangs festzulegen. Sind allerdings mehr Paare von Referenz-Messwerten bekannt, so kann eine Genauigkeit verbessert werden, da Fehler herausgemittelt werden können.

Zusammenfassend ist festzuhalten, dass basierend auf einer Mehrzahl von Paaren von Referenz-Messwerten (z.B. Referenz-Druckwerten und Referenz-Laufzeitwerten) die erste Abbildung festgelegt werden kann.

In einem zweiten Schritt der Kalibrierung kann beispielsweise ein qualitativer Verlauf der zweiten Abbildung festgelegt werden, wie dies bei Bezugszeichen 830 gezeigt ist. Die zweite Abbildung ist hier beispielsweise durch einen Kurvenlauf 831 beschrieben, der durch eine geschlossene Funktionsvorschrift, durch eine Wertetabelle oder in anderer Weise repräsentiert sein kann. In der Fig. 8a beschreibt eine Abszisse 832 einen Druck $P_{tm}$, während hingegen eine Ordinate 834 beispielsweise ein Volumen oder ein Mess-Signal (z.B. von einem Plethysmographen) beschreibt. Bei einem Ausführungsbeispiel gemäß der Erfindung wird ein qualitativer Verlauf der zweiten Abbildung basierend auf einem qualitativen Verlauf der ersten Abbildung festgelegt. Beispielsweise kann bei einigen Ausführungsbeispielen der qualitative Verlauf der zweiten Abbildung (beispielsweise ohne Verwendung weiterer Informationen) aus dem qualitativen Verlauf der ersten Abbildung bestimmt werden, indem beispielsweise die erste Abbildung bezüglich des Drucks $P_{tm}$ integriert wird. Insofern können die Abszissen 812, 832 in der gleichen Weise skaliert sein. Eine Skalierung der Ordinate 834 wird bei einigen Ausführungsbeispielen hingegen separat festgelegt und nicht unmittelbar anhand des Verlaufs der ersten Abbildung festgelegt.

Fig. 8b zeigt bei Bezugszeichen 850 eine mögliche Vorgehensweise bei einer Festlegung eines quantitativen Verlaufs (also beispielsweise einer Skalierung) der zweiten Abbildung. Die zweite Abbildung ist hier wiederum durch einen Kurvenverlauf 851 dargestellt. Eine Abszisse 852 beschreibt den Druck $P_{tm}$ und eine Ordinate 854 beschreibt ein Volumen eines elastischen Fluid-Leiters oder einen Wert eines entsprechenden Mess-Signals (z.B. des ersten Mess-Signals oder des zweiten Mess-Signals). Gemäß einem Ausführungsbeispiel werden für einen pulsierenden Fluidfluss zwei Druckwerte in verschiedenen Phasen des pulsierenden Flusses bestimmt. Beispielsweise können bei im Wesentlichen unveränderte Bedingungen des pulsierenden Flusses ein Maximalwert eines Drucks sowie ein Minimalwert des Drucks bestimmt werden. Bei einem Ausführungsbeispiel können der Maximalwert des Drucks und der Minimalwert des Drucks auf eine einzige Periode des im Wesentlichen periodisch pulsierenden Flusses bezogen sein, oder auf eine geringe Anzahl von beispielsweise maximal fünf oder zehn aufeinander folgenden Perioden des pulsierenden Flusses bezogen sein. Ein entsprechender maximaler Druckwert ist beispielsweise mit $P_{tmref1}$ bezeichnet, und ein entsprechender, zugehöriger minimaler Druckwert ist beispielsweise mit $P_{tmref1'}$ bezeichnet. Unter Verwendung des qualitativen Verlaufs der zweiten Abbildung, der ja bereits vorher bestimmt worden sein kann, wird dann beispielsweise bestimmt, welche Werte (zum Beispiel Volumenwerte V) oder welcher Differenzwert (zum Beispiel Volumendifferenz $\Delta V$)

den Druckwerten $P_{tmref1}$, $P_{tmref1'}$ gemäß der (zum Beispiel noch unskalierten) zweiten Abbildung zugeordnet werden.

Die noch unskalierte zweite Abbildung kann dann beispielsweise so skaliert werden, dass ein Unterschied zwischen einem Mess-Signal-Wert MS1, der dem Druckwert $P_{tmref1}$ durch die skalierte zweite Abbildung zugeordnet wird, und einem Mess-Signal-Wert MS1', der dem Druckwert $P_{tmref1'}$ durch die skalierte zweite Abbildung zugeordnet wird, gleich dem Amplitudenwert PA1 ist. Der Amplitudenwert PA1 ist beispielsweise ein Amplitudenwert eines tatsächlich gemessenen Mess-Signals für einen Puls, der die Druckwerte $P_{tmref1}$, $P_{tmref1'}$ aufweist.

Die Skalierung kann aber auch erfolgen, indem ein Skalierungsfaktor bestimmt wird, der beispielsweise ein Verhältnis zwischen einer Amplitude PA eines Messsignals und einer zugehörigen Volumenänderung beschreibt. Der entsprechende Skalierungsfaktor oder eine Skalierungsvorschrift, die eine Zuordnung zwischen einer Amplitude PA und einem Druckunterschied beschreibt, kann beispielsweise Teil der zweiten Abbildung sein.

Zusammenfassend ist somit festzuhalten, dass ein Verlauf der ersten Abbildung beispielsweise unter Verwendung von Referenz-Druckwerten $P_{tmref1}$, $P_{tmref2}$, $P_{tmref3}$ bestimmt werden kann, wobei die genannten Druckwerte zu unterschiedlichen Pulsen des pulsierenden Flusses gehören, wobei die unterschiedlichen Pulse des pulsierenden Flusses bei unterschiedlichen Druckverhältnissen gemessen werden. Ein qualitativer Verlauf der zweiten Abbildung wird basierend auf einem qualitativen oder quantitativen Verlauf der ersten Abbildung festgelegt, beispielsweise durch Integration der ersten Abbildung über den Druck. Eine derartige Vorgehensweise bringt mit sich, dass jeweils die charakteristischen Punkte (z.B. Maxima, Minima oder Mittelwerte) von Pulswellen ausgewertet werden können, um die erste Abbildung und den quantitativen Verlauf der zweiten Abbildung festzulegen. Die Skalierung der zweiten Abbildung hingegen erfolgt durch Auswertung einer Amplitude eines Mess-Signals für einen Puls oder für den Zeitraum, der Pulse mit zumindest näherungsweise gleichen Druckverhältnissen umfasst. Durch eine Heranziehung einer Pulsamplitude zur Skalierung der zweiten Abbildung kann beispielsweise vermieden werden, dass ein Absolutwert der Mess-Signale, der sich durch einen Gleichanteils-Drift mit der Zeit ändern kann, in die Skalierung mit einfließt. Bei einigen Ausführungsbeispielen können durch die entsprechende Skalierung der zweiten Abbildung sogar Gleichsignal-Anteile der Mess-Signale vernachlässigt bzw. unterdrückt werden, ohne dass die Messgenauigkeit wesentlich darunter leidet. Insofern ermöglichen einige Ausführungsbeispiele der Erfindung, den Druck basierend nur auf Wechselanteilen der Mess-Signale zu bestimmen.

Fig. 8c zeigt im Übrigen eine schematische Darstellung mehrerer Pulse, die beispielsweise bei einer Kalibrierung ausgewertet werden können. Die schematische Darstellung gemäß der Fig. 8c ist in ihrer Gesamtheit mit 870 bezeichnet. Eine Abszisse 872 beschreibt eine Zeit in willkürlichen Einheiten, und eine Ordinate 874 beschreibt einen Druck $P_{tm}$. Ein erster Puls 880 erfolgt beispielsweise auf einem hohen Druckniveau, ein zweiter Puls 882 erfolgt beispielsweise auf einem mittleren Druckniveau, und ein dritter Puls 884 erfolgt beispielsweise auf einem niedrigen Druckniveau. Entsprechende Druckwerte sind in der Fig. 8c entsprechend bezeichnet.

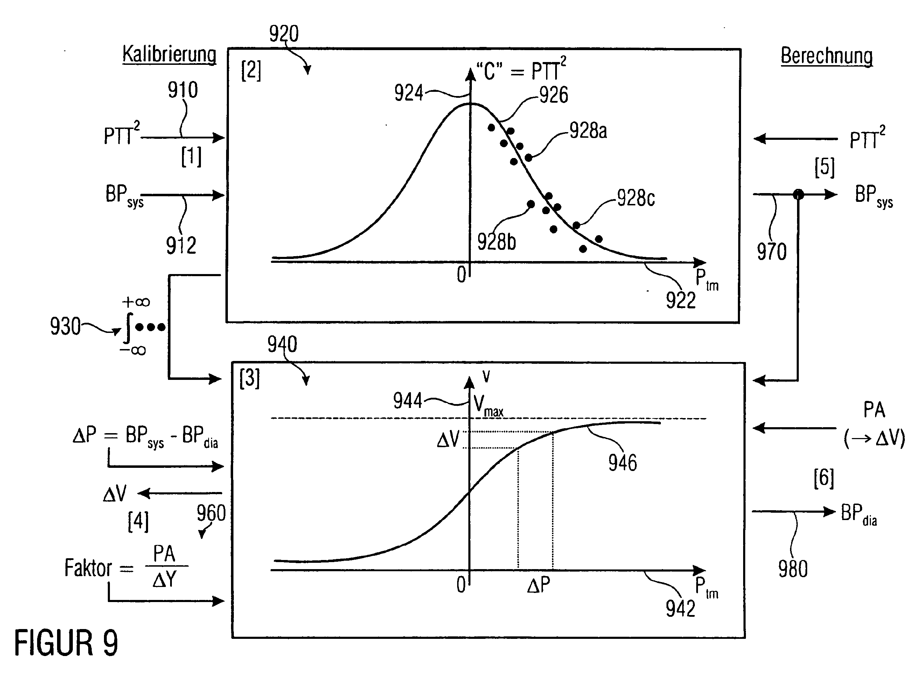

Im Folgenden wird ein weiteres Ausführungsbeispiel gemäß der Erfindung bezugnehmend auf die Fig. 9 kurz erläutert. Die Fig. 9 zeigt eine Übersicht über einzelne Komponenten eines Blutdruckmodells und über einzelne Signalverarbeitungsschritte.

Ein System gemäß einem Ausführungsbeispiel der Erfindung beruht in seiner Anwendung beispielsweise zur nicht-invasiven, kontinuierlichen Blutdruckmessung ohne Manschette auf den Parametern Pulswellen-Laufzeit (PTT) und Puls-Amplitude (PA).

Beide Parameter basieren dabei wiederum beispielsweise auf einer Zeit-synchronen Ableitung von Plethysmogrammen zweier voneinander entfernter Plethysmographen, die beispielsweise an einem Unterarm eines Patienten angebracht sind. Eine Puls-Amplitude kann dabei beispielsweise für jeden Herzschlag (Schlag-für-Schlag bzw. "beat-by-beat") aus einem der beiden Plethysmogramme ermittelt werden. Die Pulswellen-Laufzeit kann beispielsweise als zeitliche Differenz zwischen Maxima (oder anderen prominenten bzw. ausgezeichneten Punkten) der beiden zeitversetzten Plethysmographen-Pulswellen berechnet oder bestimmt werden. Details diesbezüglich sind beispielsweise den Figuren 3 und 4 entnehmbar.

Die Parameter Pulswellen-Laufzeit (PTT) und Puls-Amplitude (PA) werden bei diesem Verfahren beispielsweise anhand eines Gefäßmodells auf einen systolischen Blutdruck und/oder auf einen diastolischen Blutdruck abgebildet, wobei bestimmte physiologische Gesetzmäßigkeiten mit berücksichtigt werden können. So ist hier beispielsweise ein Verhältnis zwischen einem Volumen V und einem entsprechenden transmuralen Druck $P_{tm}$ eines betrachteten Gefäßabschnitts durch sein individuelles Druck-Volumen-Diagramm gegeben, welches sich beispielsweise sehr gut als Sigmoid-funktion des Volumens über dem Druck darstellen lässt.

Bei einer Steigung des Druck-Volumen-Diagramms (bzw. einer Kurve, die einen Zusammenhang zwischen Druck und Volumen beschreibt) handelt es sich beispielsweise um eine sogenannte "Compliance" (elastische Nachgiebigkeit), also um ein Maß für eine Dehnbarkeit des Gefäßes (bzw. des Blutgefäßes, bzw. des Fluidleiters), woraus sich ein Druck-Compliance-Diagramm (bzw. Druck-Nachgiebigkeits-Diagramm) als erste Ableitung des Druck-Volumen-Diagramms ergibt. Im Übrigen spielt außerdem die sogenannte "Gleichung von Bramwell und Hill" - eine Modifikation einer Moens-Korteweg-Gleichung aus der Strömungsmechanik - noch eine wichtige Rolle, da sie einen Zusammenhang zwischen einer Pulswellen-Geschwindigkeit und einem Elastizitätsmodul eines Rohres (oder Fluidleiters) und somit einen Zusammenhang zwischen der Pulswellen-Laufzeit und der Compliance C (bzw. Nachgiebigkeit C) eines Gefäßabschnitts beschreibt. Beispielsweise gilt zumindest näherungsweise der folgende Zusammenhang zwischen dem quadrierten Laufzeitwert $PTT^2$ und der Nachgiebigkeit bzw. Compliance C:

$$PTT^2 = const. * C,$$

wobei "const." Beispielsweise eine Konstante bezeichnet.

Vor einer Berechnung eines Blutdruck-Äquivalents anhand der Pulswellen-Laufzeit PTT und der Puls-Amplitude PA wird beispielsweise ein Gefäßmodell individuell an den Patienten angepasst und kalibriert, indem verschiedene systolische und diastolische Blutdruckwerte (z.B. auskultatorisch oder oszillometrisch) zusammen mit ihren korrespondierenden Pulswellen-Laufzeiten und Puls-Amplituden gemessen werden. Aus den systolischen Blutdruckwerten und den zugehörigen Compliance-Werten bzw. Nachgiebigkeits-Werten bzw. $PTT^2$-Werten (in der schematischen Darstellung der Fig. 9 mit 910, 912 bzw. mit [1] bezeichnet) lässt sich nun, beispielsweise mittels einer Methode der kleinsten Fehlerquadrate (auch als "least-mean-squares"-Methode bekannt) eine dem (betrachteten) Gefäßabschnitt zugrunde liegende, zumindest näherungsweise Gauß-förmige Druck-Compliance-Ersatz-Kurve bzw. Druck-Nachgiebigkeit-Ersatz-Kurve approximieren (in der Fig. 9 mit [2] bzw. 920 bezeichnet).

Die Druck-Compliance-Ersatz-Kurve bzw. Druck-Nachgiebigkeit-Ersatz-Kurve ist in der Fig. 9 in einem Koordinatensystem mit einer Abszisse 922, die den Druck $P_{tm}$ beschreibt, und einer Ordinate 924, die Nachgiebigkeit bzw. Compliance "C" oder ein Quadrat der Pulswellenlaufzeit $PTT^2$ beschreibt, dargestellt. Die Druck-Compliance-Ersatz-Kurve ist beispielsweise durch einen oder mehrere Parameter beschrieben, die beispielsweise im Rahmen der Kalibrierung so festgelegt werden, dass die Druck-Compliance-Ersatz-Kurve eine Mehrzahl von Paaren von systolischen Blutdruckwerten und zugehörigen Compliance-Werten, die durch Punkte 928a, 928b, 928c gezeigt sind, ausreichend gut annähert.

Durch eine Integration (z.B. durch eine Integration über den Druck $P_{tm}$) der Gauß-förmigen Druck-Compliance-Ersatz-Kurve (die beispielsweise bei Bezugszeichen 930 symbolisch dargestellt ist) besteht beispielsweise in einem nächsten Schritt eine entsprechende sigmoidale Druck-Volumen-Ersatz-Kurve 940 (auch mit [3] bezeichnet).

Eine sigmoidale Kurve bzw. Sigmoid-Funktion, auch als "Schwanenhals-Funktion" oder "S-shaped function" bezeichnet, ist beispielsweise eine mathematische Funktion mit einem S-förmigen Funktionsverlauf. Im allgemeinen ist eine Sigmoid-Funktion beispielsweise eine beschränkte und differenzierbare reelle Funktion mit einer positiven oder negativen ersten Ableitung und genau einem Wendepunkt. Beispielsweise ist das Integral einer glatten, positiven Funktion mit einem "Berg" (z.B. der Gauß-schen Glocken-Kurve) wiederum eine Sigmoid-Funktion.

Im Hinblick auf die Druck-Volumen-Ersatz-Kurve ist festzuhalten, dass diese in der Fig. 9 in einem Koordinatensystem mit einer Abszisse 942, die den Druck $P_{tm}$ beschreibt, und einer Ordinate 944, die beispielsweise ein lokales Volumen eines Blutgefäßes oder eines elastischen Fluidleiters beschreibt, durch eine Kurve 946 dargestellt ist. Eine untere Schranke der Druck-Volumen-Ersatz-Kurve liegt beispielsweise bei V = 0, und ein Maximum der Druck-Volumen-Ersatz-Kurve liegt beispielsweise bei V = $V_{max}$.

Die Druck-Volumen-Ersatz-Kurve ermöglicht es beispielsweise, einer Volumenveränderung ΔV eine Druckänderung ΔP zuzuordnen und/oder umgekehrt. Der Zusammenhang zwischen der Volumenänderung ΔV und der Druckänderung ΔP ist dabei im Übrigen aufgrund der Nichtlinearität der Druck-Volumen-Ersatz-Kurve druckabhängig.

Mit Hilfe eines systolisch-diastolischen Blutdruckwertpaars und seiner Differenz, dem sogenannten Pulsdruck ΔP, kann schließlich noch ein Verhältnis (beispielsweise mit [4] bzw. 960 bezeichnet) zwischen der zugehörigen gemessenen Puls-Amplitude und der entsprechenden Volumendifferenz ΔV in dem Druck-Volumen-Ersatz-Diagramm ermittelt werden, um beispielsweise später (z.B. bei einer Druckmessung bzw. Blutdruckmessung) die Puls-Amplitude (z.B. eine Amplitude PA des Mess-Signals) auf eine Volumendifferenz ΔV abbilden zu können.

Die Berechnung beispielsweise eines systolischen und diastolischen Blutdruck-Äquivalents mit Hilfe des individuell kalibrierten Modells kann nun beispielsweise anhand der Schlag-für-Schlag ("beat-by-beat") gemessenen Pulswellen-Laufzeit und Puls-Amplitude erfolgen. Das PTT[2] (also beispielsweise ein Quadrat der Pulswellen-Laufzeit) wird dabei beispielsweise über das Druck-Compliance-Ersatz-Diagramm ("Druck-Nachgiebigkeit-Ersatz-Diagramm") auf den systolischen Blutdruckwert abgebildet, wie beispielsweise durch [5] bzw. Bezugszeichen 970 angedeutet ist. Dieser (also beispielsweise der systolische Blutdruckwert) wiederum ermöglicht zusammen mit der Puls-Amplitude (bzw. der systo-diastolischen Volumendifferenz ΔV) über das Druck-Volumen-Ersatz-Diagramm eine Berechnung der systo-diastolischen Druckdifferenz ΔP und somit auch eine Berechnung des diastolischen Blutdruckwerts, wie beispielsweise durch [6] bzw. Bezugszeichen 980 angedeutet ist. In anderen Worten, ein Unterschied zwischen dem systolischen Blutdruckwert und dem diastolischen Blutdruckwert kann beispielsweise als Druck-amplitude bezeichnet werden.

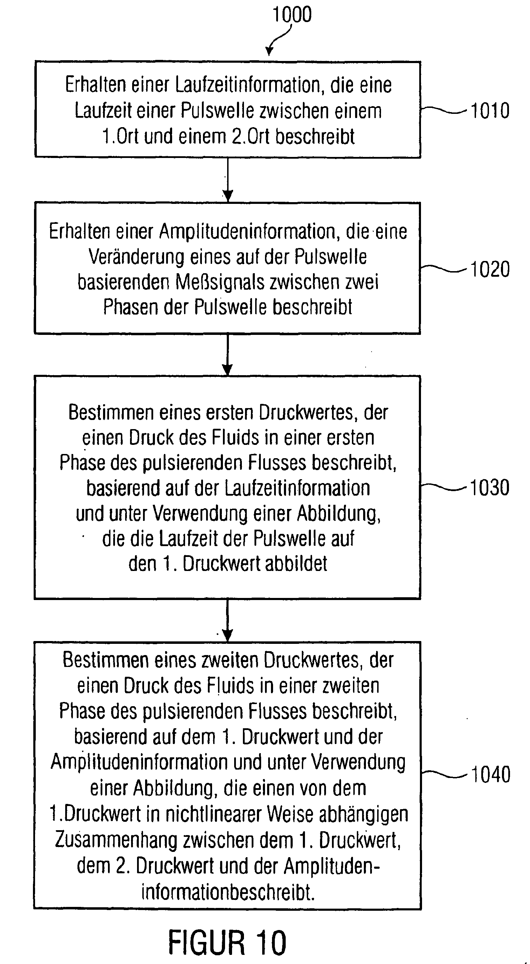

Fig. 10 zeigt ein Flussdiagramm eines Verfahrens zum Bestimmen von zwei Druckwerten, gemäß einem Ausführungsbeispiel der Erfindung. Das Verfahren gemäß der Fig. 10 ist in seiner Gesamtheit mit 1000 bezeichnet. Das Verfahren 1000 umfasst ein Erhalten 1010 einer Laufzeitinformation, die eine Laufzeit einer Pulswelle zwischen einem ersten Ort und einem zweiten Ort beschreibt. Das Verfahren umfasst ferner ein Erhalten 1020 einer Amplitudeninformation, die eine Veränderung eines auf der Pulswelle basierenden Mess-Signals zwischen zwei Phasen der Pulswelle beschreibt. Eine erste Phase der Pulswelle kann beispielsweise ein Maximum (zum Beispiel im Hinblick auf einen lokalen Druck oder im Hinblick auf eine lokale Ausdehnung eines Fluidleiters oder eines Blutgefäßes) sein, und eine zweite Phase der Pulswelle kann beispielsweise eine Ruhephase sein, oder umgekehrt. Das Verfahren 1000 umfasst ein Bestimmen 1030 eines ersten Druckwertes, der einen Druck des Fluids in einer ersten Phase des pulsierenden Flusses beschreibt, basierend auf der Laufzeitinformation und unter Verwendung einer ersten Abbildung, die eine Laufzeit der Pulswelle auf den ersten Druckwert abbildet. Das Verfahren 1000 umfasst ein Bestimmen 1040 eines zweiten Druckwerts, der einen Druck des Fluids in einer zweiten Phase des pulsierenden Flusses beschreibt, basierend auf dem ersten Druckwert und der Amplitudeninformation, unter Verwendung einer zweiten Abbildung, die einen von dem ersten Druckwert in nichtlinearer Weise abhängigen Zusammenhang zwischen dem ersten Druckwert, dem zweiten Druckwert und der Amplitudeninformation beschreibt.

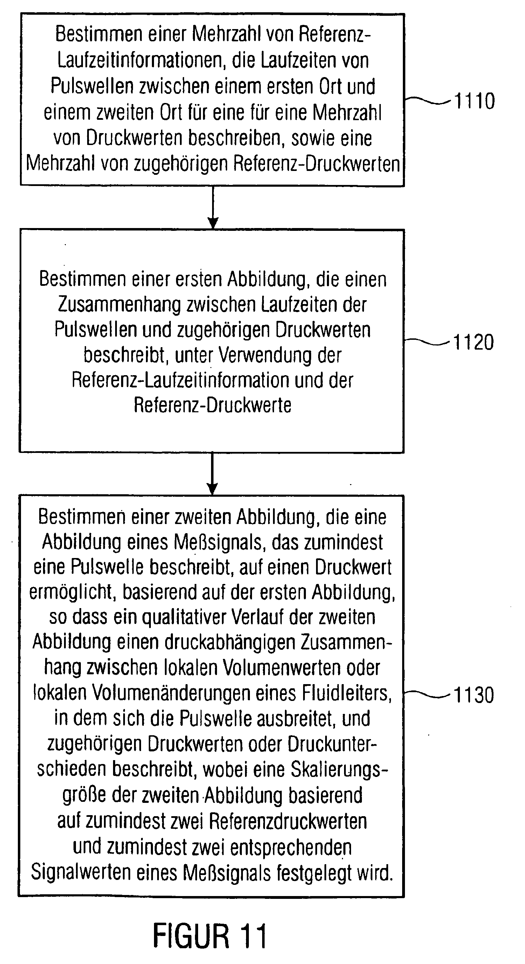

Fig. 11 zeigt ein Flussdiagramm eines Verfahrens zum Kalibrieren eine Druckmessers, gemäß dem Ausführungsbeispiel der Erfindung. Das Verfahren gemäß der Fig. 11 ist in seiner Gesamtheit mit 1100 bezeichnet.

Das Verfahren 1100 umfasst ein Bestimmen 1110 einer Mehrzahl von Referenz-Laufzeit-Informationen, die Laufzeiten von Pulswellen zwischen einem ersten Ort und einem zweiten Ort für eine Mehrzahl von Druckwerten beschreiben, sowie einer Mehrzahl von zugehörigen Referenz-Druckwerten. Das Verfahren 1100 umfasst ein Bestimmen 1120 einer ersten Abbildung, die einen Zusammenhang zwischen Laufzeiten der Pulswellen und zugehörigen Druckwerten beschreibt, unter Verwendung der Referenz-Laufzeitinformationen und der Referenz-Druckwerte. Das Verfahren 1100 umfasst zudem ein Bestimmen 1130 einer zweiten Abbildung, die eine Abbildung einer Amplitudeninformation eines Mess-Signals, das zumindest eine Pulswelle beschreibt, auf einen Druckwert ermöglicht, basierend auf der ersten Abbildung. Das Bestimmen der zweiten Abbildung erfolgt derart, dass ein qualitativer Verlauf der zweiten Abbildung einen druckabhängigen Zusammenhang zwischen lokalen Volumenwerten oder lokalen Volumenänderungen eines Fluidleiters, in dem sich die Pulswelle ausbreitet, und zugehörigen Druckwerten oder Druckunterschieden beschreibt. Das Bestimmen der zweiten Abbildung erfolgt im Übrigen derart, dass eine Skalierungsgröße der zweiten Abbildung basierend auf zumindest zwei Referenz-druckwerten und zumindest zwei entsprechenden Signalwerten eines Mess-Signals festgelegt wird.

Zusammenfassend ist somit festzuhalten, dass einige Ausführungsbeispiele gemäß der Erfindung die folgenden charakteristischen Merkmale aufweisen:

• Nichtinvasive, kontinuierliche (Schlag-für-Schlag bzw. "beat-by-beat") Abschätzung eines arteriellen Blutdrucks ohne Manschette nach einmaliger auskultatorischer oder oszillometrischer Kalibrierung des Systems auf eine individuelle Gefäß-Physiologie; und

• Betrachtung eines homogenen, elastischen Gefäßabschnitts auf Basis der beiden Plethysmographen am Unterarm (wie beispielsweise in Fig. 3 gezeigt) und damit Ausschluss einer elektro-mechanischen Latenzzeit des Herzens und der aktiven Einflüsse der Gefäßmuskulatur (z.B. der Arteriolen) auf eine periphere Durchblutungsregulation.

Zusammenfassend ist festzuhalten, dass einige der hierin beschriebenen Ausführungsbeispiele bzw. einige Ausführungsbeispiele des hierin beschriebenen Systems einer nicht-invasiven, kontinuierlichen und belastungsfreien (ohne Manschette) Blutdruckabschätzung auf Grundlage der Pulswellenlaufzeit und der Pulswellen-Amplitude dienen. Diese Parameter (also beispielsweise Pulswellen-Laufzeit und Pulswellen-Amplitude) werden beispielsweise mittels zweier Plethysmographen am Unterarm auf einem elastischen und bezüglich der Gefäßanatomie homogenen Gefäßabschnitt ("arteria ulnaris" bzw. Ellenarterie und "arteria radialis" bzw. Speichenarterie) gemessen. Die elektromechanische Latenzzeit des Herzens sowie die peripheren Regulationsvorgänge als Fehlerquelle werden auf diese Weise umgangen. Eine einmalige Kalibrierung des zugrunde liegenden Modells und eine daraufhin mögliche Berechnung des Blutdruck-Äquivalents geschehen unter Berücksichtung physiologischer Gesetzmäßigkeiten wie den Druck-Volumen-Verhältnissen, der Compliance (Nachgiebigkeit bzw. Dehnbarkeit) und der Pulswellen-Geschwindigkeit des betrachteten Gefäßabschnitts.

Zusammenfassend kann ferner festgehalten werden, dass das hierin beschriebene Konzept bzw. Verfahren beispielsweise auf dem Gebiet der nicht-invasiven, kontinuierlichen Blutdruckmessung Anwendung findet, wobei es grundlegende Prinzipien der Gefäß-Physiologie ausnutzt, um Beziehungen zwischen dem systolischen/diastolischen Blutdruck und den Plethysmographie-basierenden Messgrößen Pulswellen-Laufzeit und Pulswellen-Amplitude herzustellen und den arteriellen Blutdruck somit abzuschätzen.

Bei der Plethysmographie handelt es sich im allgemeinen um ein nicht-invasives Messverfahren zur Erfassung von Volumenänderungen. In diesem Anwendungsgebiet wird dieses Verfahren angewandt, um spezielle Pulswellen-bedingte Volumenänderungen arterieller Gefäßabschnitte zu messen. Grob unterschieden werden können hier beispielsweise eine Volumen-Plethysmographie, eine Foto-Plethysmographie, eine Impedanz-Plethysmographie und eine kapazitive Plethysmographie.

Im Folgenden wird noch kurz aufgeführt, inwiefern einige Ausführungsbeispiele eine Verbesserung gegenüber herkömmlichen Systemen bzw. Verfahren darstellen. Bisherige bzw. herkömmliche Verfahren zur nichtinvasiven Abschätzung des Parameters "Blutdruck" beruhen größtenteils auf einer negativen Korrelation zwischen der Pulswellen-Laufzeit (PTT) und dem arteriellen Blutdruck (BP) und auch auf der Pulswellen-Amplitude. Zur Erfassung dieser Pulswellen-Parameter wird üblicherweise ein QRS-Komplex in einem Elektrokardiogramm (EKG) als Startzeitpunkt und ein Maximum in einem peripheren Foto-Plethysmogramm (z.B. am Finger oder am Ohr) als Ankunftszeitpunkt der Pulswelle ausgewählt.

Eine elektromechanische Latenzzeit des Herzens, die zwischen der maximalen Myokarderregung (QRS-Komplex) und dem eigentlichen Blutauswurf aus der linken Herzkammer liegt, wirkt sich dabei verfälschend auf die zu berechnende Pulswellen-Laufzeit (PTT) aus, da sie nicht bei allen Patienten als konstant angenommen werden kann. Des Weiteren wird über die (von zentral zu peripher) sich stetig ändernden passiven und aktiven Eigenschaften des arteriellen Gefäßsystems z.B. zur Durchblutungsregulation in der Peripherie gemittelt, wodurch nur vage Rückschlüsse auf die zentralen Verhältnisse bezüglich Pulswellen-Laufzeit und PulsAmplitude gemacht werden können.

Zur Berechnung eines Blutdruck-Äquivalents anhand der Pulswellen-Laufzeit (und Pulswellen-Amplitude) existieren bereits zahlreiche lineare und nichtlineare Modelle, die in der Mehrzahl rein empirisch begründet sind und keinen physiologischen Hintergrund haben. Bei einigen Ausführungsbeispielen gemäß der Erfindung wird durch das oben beschriebene physiologisch basierte Modell, das sich beispielsweise in der ersten Abbildung und der zweiten Abbildung wiederspiegelt, eine besonders präzise und/oder zuverlässige Bestimmung von Druckwerten bzw. Blutdruckwerten ermöglicht.

Zusammenfassend ist festzuhalten, dass hierin verschiedene Ausführungsbeispiele eines Druckmessers bzw. eines Blutdruckmessers beschrieben wurden. Ganz allgemein ist festzuhalten, dass Ausführungsbeispiele, die anhand der Blutdruckmessung beschrieben wurden, auch für eine allgemeine Druckmessung eingesetzt werden können. Insofern kann ein Blutdruckmesser auch immer ganz allgemein als ein Druckmesser angesehen werden. Auf einen Blutdruck bezogene Werte können im Übrigen auch ganz allgemein als auf einen Druck bezogene Werte angesehen werden.

Die hierin beschriebenen Verfahren können auf verschiedene Weise realisiert werden. Bei einigen Ausführungsbeispielen kann ein Computerprogramm verwendet werden.

In anderen Worten, die erfindungsgemäßen Vorrichtungen und die erfindungsgemäßen Verfahren können in Hardware oder in Software implementiert werden. Die Implementation kann auf einem digitalen Speichermedium, beispielsweise einer Diskette, einer CD, einer DVD, einem ROM, einem PROM, einem EPROM, einem EEPROM, oder einem FLASH-Speicher, mit elektronisch auslesbaren Steuersignalen erfolgen, die so mit einem programmierbaren Computersystem zusammenwirken können, dass das entsprechende Verfahren ausgeführt wird. Allgemein besteht die Erfindung somit auch in einem Computer-Programm-Produkt mit auf einem maschinenlesbaren Träger gespeicherten Programmcode zur Durchführung des erfindungsgemäßen Verfahrens, wenn das Computerprogramm-Produkt auf einem Rechner abläuft. In anderen Worten ausgedrückt, die Erfindung kann als ein Computer-Programm mit einem Programmcode zur Durchführung des erfindungsgemäßen Verfahrens realisiert werden, wenn das Computer-Programm auf einem Computer abläuft.

Ein Ausführungsbeispiel der Erfindung schafft einen Druckmesser 100; 700 zum Bestimmen zumindest eines Druckwerts 122, der einen Druck eines in pulsierender Weise fließenden Fluids in einer Phase des pulsierenden Flusses beschreibt, mit folgenden Merkmalen: einem Pulswellen-Charakterisierer 110, der ausgelegt ist, um eine Laufzeitinformation 112, die eine Laufzeit einer Pulswelle zwischen einem ersten Ort und einem zweiten Ort beschreibt, und eine Amplitudeninformation 114, die eine Veränderung eines auf der Pulswelle basierenden Mess-Signals zwischen zwei Phasen der Pulswelle beschreibt, zu erhalten; und einem Druckwertbestimmer 120, der ausgelegt ist, um einen ersten Druckwert 132, der einen Druck des Fluids in einer ersten Phase des pulsierenden Flusses beschreibt, basierend auf der Laufzeitinformation 112 und unter Verwendung einer ersten Abbildung 134, die die Laufzeit der Pulswelle auf den ersten Druckwert abbildet, zu erhalten, und um einem zweiten Druckwert 136, der einen Druck des Fluids in einer zweiten Phase des pulsierenden Flusses beschreibt, basierend auf dem ersten Druckwert 132 und der Amplitudeninformation 114 unter Verwendung einer zweiten Abbildung 138 zu erhalten, wobei die zweite Abbildung einen von dem ersten Druckwert 132 abhängigen Zusammenhang zwischen dem ersten Druckwert 132, dem zweiten Druckwert 136 und der Amplitudeninformation 114 beschreibt.

Bei einem bevorzugten Ausführungsbeispiel des Druckmessers ist der Pulswellencharakterisierer 110 ausgelegt, um ein erstes Plethysmographen-Signal 710 und ein zweites Plethysmographen-Signal 714 zu empfangen, und um die Laufzeitinformation 112 basierend auf dem ersten Plethysmographen-Signal und dem zweiten Plethysmographen-Signal zu erzeugen.

Bei einem bevorzugten Ausführungsbeispiel des Druckmessers 100;700 ist der Pulswellen-Charakterisierer 110 ausgelegt, um die Amplitudeninformation 114 basierend auf einem Plethysmographen-Signal zu erzeugen, so dass die Amplitudeninformation zumindest näherungsweise eine Amplitude des Plethysmographen-Signals beschreibt.

Bei einem bevorzugten Ausführungsbeispiel des Druckmessers 100;700 weist der Druckmesser zumindest einen Plethysmographen 712, 716 auf, der ausgelegt ist, um ein Plethysmographen-Signal 710, 714 zu liefern, das einen zeitlichen Verlauf einer Durchblutung eines Blutgefäßes oder einen zeitlichen Verlauf einer Volumenänderung eines Fluidleiters beschreibt.

Bei einem bevorzugten Ausführungsbeispiel des Druckmessers 100;700 weist der Druckmesser zumindest zwei Plethysmographen 712, 716 auf, wobei ein erster Plethysmograph 712 ausgelegt ist, um ein erstes Plethysmographen-Signal 710 zu liefern, das einen zeitlichen Verlauf einer Durchblutung eines Blutgefäßes an einem ersten Ort beschreibt, und wobei ein zweiter Plethysmograph 716 ausgelegt ist, um ein zweites Plethysmographen-Signal 714 zu liefern, das einen zeitlichen Verlauf einer Durchblutung eines Blutgefäßes an einem zweiten Ort beschreibt.

Bei einem bevorzugten Ausführungsbeispiel des Druckmessers 100;700 sind der erste Plethysmograph und der zweite Plethysmograph ausgelegt, um an einem gemeinsamen, im Hinblick auf eine Gefäßanatomie homogenen Gefäßabschnitt angebracht zu werden, und um Mess-Signale zu liefern, die eine Ausbreitung einer Pulswelle in dem Gefäßabschnitt beschreiben.

Bei einem bevorzugten Ausführungsbeispiel des Druckmessers 100;700 sind der erste Plethysmograph 712 und der zweite Plethysmograph 716 ausgelegt, um gemeinsam in einem vorgegebenen Abstand an einem Oberarm, an einem Unterarm, an einem Oberschenkel oder an einem Unterschenkel angebracht zu werden.

Bei einem bevorzugten Ausführungsbeispiel des Druckmessers 100;700 ist der Druckmesser ausgelegt, um eine Abbildungsvorschrift 530; 850; 940, die die zweite Abbildung 138 beschreibt, durch Integration einer Abbildungsvorschrift 520; 810; 920, die die erste Abbildung 134 beschreibt, zu erhalten.

Bei einem bevorzugten Ausführungsbeispiel des Druckmessers 100;700 ist der Druckmesser ausgelegt um die erste Abbildung 134 und die zweite Abbildung 138 basierend auf einer Mehrzahl von einander zugeordneten Referenz-Laufzeitinformationen, Referenz-Amplitudeninformationen und Referenz-Druckwerten zu kalibrieren.

Ein Ausführungsbeispiel der Erfindung schafft ein Verfahren 1000 zum Bestimmen von Druckwerten, die einen Druck eines in pulsierender Weise fließenden Fluids in zumindest zwei Phasen des pulsierenden Flusses beschreiben, unter Verwendung eines Druckmesser 100, mit folgenden Schritten: Erhalten 1010 einer Laufzeitinformation, die eine Laufzeit einer Pulswelle zwischen einem ersten Ort und einem zweiten Ort beschreibt; Erhalten 1020 einer Amplitudeninformation, die eine Veränderung eines auf der Pulswelle basierenden Mess-Signals zwischen zwei Phasen der Pulswelle beschreibt; Bestimmen 1030 eines ersten Druckwerts, der einen Druck des Fluids in einer ersten Phase des pulsierenden Flusses beschreibt, basierend auf der Laufzeitinformation und unter Verwendung einer ersten Abbildung, die eine Laufzeit der Pulswelle auf den ersten Druckwert abbildet; und Bestimmen 1040 eines zweiten Druckwerts, der einen Druck des Fluids in einer zweiten Phase des pulsierenden Flusses beschreibt, basierend auf dem ersten Druckwert und der Amplitudeninformation unter Verwendung einer zweiten Abbildung, die einen von dem ersten Druckwert abhängigen Zusammenhang zwischen dem ersten Druckwert, dem zweiten Druckwert und der Amplitudeninformation beschreibt.

Bei einem bevorzugten Ausführungsbeispiel des Verfahrens beschreibt die zweite Abbildung einen in nichtlinearer Weise von dem ersten Druckwert abhängigen Zusammenhang zwischen dem ersten Druckwert, dem zweiten Druckwert und der Amplitudeninformation.

Ein Ausführungsbeispiel der Erfindung schafft ein Verfahren 1100 zum Kalibrieren eines Druckmessers, der ausgelegt ist, um zumindest einen Druckwert 122, der einen Druck eines in pulsierender Weise fließenden Fluids in einer Phase des pulsierenden Flusses beschreibt, unter Verwendung einer ersten Abbildung und einer zweiten Abbildung zu bestimmen, mit folgenden Schritten: Bestimmen 1110 einer Mehrzahl von Referenz-Laufzeitinformationen, die Laufzeiten von Pulswellen zwischen einem ersten Ort und einem zweiten Ort für eine Mehrzahl von Druckwerten beschreiben, sowie einer Mehrzahl von zugehörigen Referenz-Druckwerten; Bestimmen 1120 der ersten Abbildung, die einen Zusammenhang zwischen Laufzeiten der Pulswellen und zugehörigen Druckwerten beschreibt, unter Verwendung der Referenz-Laufzeitinformationen und der Referenz-Druckwerte; und Bestimmen 1130 der zweiten Abbildung, die einen von einem ersten Druckwert abhängigen Zusammenhang zwischen dem ersten Druckwert, einem zweiten Druckwert und einer Amplitudeninformation beschreibt, wobei ein qualitativer Verlauf der zweiten Abbildung basierend auf der ersten Abbildung festgelegt wird.

Bei einem bevorzugten Ausführungsbeispiel des Verfahrens 1100 wird der qualitative Verlauf der zweiten Abbildung durch Integration eines Verlaufs der ersten Abbildung festgelegt.

Bei einem bevorzugten Ausführungsbeispiel des Verfahrens 1100 ist ein Abbildungsparameter, der die zweite Abbildung beschreibt, gleich einem Abbildungsparameter (b), der die erste Abbildung beschreibt, oder ein Abbildungsparameter, der die zweite Abbildung beschreibt, wird von einem Abbildungsparameter (b), der die erste Abbildung beschreibt, abgeleitet.

Bei einem bevorzugten Ausführungsbeispiel des Verfahrens beschreibt der Abbildungsparameter eine Druck-Skalierung.

Bei einem bevorzugten Ausführungsbeispiel des Verfahrens wird die zweite Abbildung so bestimmt, dass ein qualitativer Verlauf der zweiten Abbildung einen druckabhängigen Zusammenhang zwischen lokalen Volumenwerten oder lokalen Volumenänderungen eines Fluidleiters, in dem sich die Pulswelle ausbreitet, und zugehörigen Druckwerten oder Druckunterschieden beschreibt.

Bei einem bevorzugten Ausführungsbeispiel des Verfahrens wird die zweite Abbildung so bestimmt, dass die zweite Abbildung einen Zusammenhang zwischen zwei zu einer Pulswelle gehörigen Druckwerten und einer Amplitude eines durch die Pulswelle erzeugten Messsignals beschreibt.

Bei einem bevorzugten Ausführungsbeispiel des Verfahrens wird eine Skalierungsgröße der zweiten Abbildung basierend auf zumindest zwei Referenz-Druckwerten und zumindest zwei entsprechenden Signal-Werten eines Mess-Signals festgelegt.

Ein Ausführungsbeispiel der Erfindung schafft ein Computer-programm zur Durchführung eines erfindungsgemäßen Verfahrens, wenn das Verfahren auf einem Computer ausgeführt wird.

**Patentansprüche**

1. Druckmesser (100; 700) zum Bestimmen zumindest eines Druckwerts (122), der einen Druck eines in pulsierender Weise fließenden Fluids in einer Phase des pulsierenden Flusses beschreibt, mit folgenden Merkmalen:

  einem Pulswellen-Charakterisierer (110), der ausgelegt ist, um eine Laufzeitinformation (112), die eine Laufzeit einer Pulswelle zwischen einem ersten Ort und einem zweiten Ort beschreibt, und eine Amplitudeninformation (114), die eine Veränderung eines auf der Pulswelle basierenden Mess-Signals zwischen zwei Phasen der Pulswelle beschreibt, zu erhalten; und einem Druckwertbestimmer (120), der ausgelegt ist, um einen ersten Druckwert (132), der einen Druck des Fluids in einer ersten Phase des pulsierenden Flusses beschreibt, basierend auf der Laufzeitinformation (112) und unter Verwendung einer ersten Abbildung (134), die die Laufzeit der Pulswelle auf den ersten Druckwert abbildet, zu erhalten, und um einem zweiten Druckwert (136), der einen Druck des Fluids in einer zweiten Phase des pulsierenden Flusses beschreibt, basierend auf dem ersten Druckwert (132) und der Amplitudeninformation (114) unter Verwendung einer zweiten Abbildung (138) zu erhalten, wobei die zweite Abbildung einen von dem ersten Druckwert (132) abhängigen Zusammenhang zwischen dem ersten Druckwert (132), dem zweiten Druckwert (136) und der Amplitudeninformation (114) beschreibt.

**2.** Druckmesser (100; 700) gemäß Anspruch 1, wobei der Druckmesser ein Blutdruckmesser zum Bestimmen eines systolischen Blutdruckwerts oder eines diastolischen Blutdruckwertes ist;
wobei der Pulswellen-Charakterisierer ausgebildet ist, um die Amplitudeninformation so zu erhalten, dass die Amplitudeninformation die Amplitude eines auf der Pulswelle basierenden Mess-Signals beschreibt;
wobei der Druckwertbestimmer ein Blutdruckwertbestimmer ist,
der ausgelegt ist, um als ersten Druckwert einen ersten Blutdruckwert basierend auf der Laufzeitinformation und unter Verwendung der ersten Abbildung, die die Laufzeit der Pulswelle auf den ersten Blutdruckwert abbildet, zu erhalten, und
der ausgelegt ist, um als zweiten Druckwert einen zweiten Blutdruckwert basierend auf dem ersten Blutdruckwert und der Amplitudeninformation unter Verwendung der zweiten Abbildung zu erhalten;
wobei die zweite Abbildung einen von dem ersten Blutdruckwert abhängigen Zusammenhang zwischen der Amplitudeninformation und einer Druck-Amplitude einer durch das Mess-Signal beschriebenen Pulswelle beschreibt; und
wobei der zweite Blutdruckwert den systolischen Blutdruckwert oder den diastolischen Blutdruckwert darstellt.

**3.** Druckmesser (100;700) gemäß Anspruch 1 oder 2, wobei der Druckwertbestimmer (120) ausgelegt ist, um bei der Bestimmung des ersten Druckwerts (132) eine erste Abbildungsvorschrift (520; 810) auszuwerten, die einen Zusammenhang zwischen einem Blutdruck ($P_{tm}$) und einer Dehnbarkeit (C) eines Blutgefäßes zumindest näherungsweise beschreibt.

**4.** Druckmesser (100;700) gemäß Anspruch 3, wobei der Druckwertbestimmer (120) ausgelegt ist, um als erste Abbildungsvorschrift (134; 520; 810), die einen Zusammenhang zwischen einem Blutdruck ($P_{tm}$) und einer Dehnbarkeit (C) eines Blutgefäßes zumindest näherungsweise beschreibt, eine Gaußkurvenartige Abbildungsvorschrift auszuwerten.

**5.** Druckmesser (100;700) gemäß Anspruch 3 oder 4, wobei der Druckwertbestimmer (120) ausgelegt ist, um bei der Bestimmung des ersten Druckwerts (132) einen Laufzeitwert (PTT), der durch die Laufzeitinformation (122) beschrieben wird, zu quadrieren, und um basierend auf dem quadrierten Laufzeitwert unter Verwendung der ersten Abbildungsvorschrift (134; 520; 810), die den Zusammenhang zwischen einem Blutdruckwert ($P_{tm}$) und einer Dehnbarkeit (C) eines Blutgefäßes beschreibt, den ersten Blutdruckwert (132) zu erhalten.

**6.** Druckmesser (100;700) gemäß einem der Ansprüche 1 bis 5, wobei der Druckwertbestimmer (120) ausgelegt ist, um bei der Bestimmung des zweiten Druckwerts (136) eine zweite Abbildungsvorschrift (138; 530; 850; 940) auszuwerten, die einen Zusammenhang zwischen einem Druckwert oder einem Druckunterschied und einem Volumenwert oder einer Volumenänderung eines elastischen Fluidleiters oder eines Blutgefäßes beschreibt.

**7.** Druckmesser (100;700) gemäß Anspruch 6, wobei der Druckwertbestimmer (120) ausgelegt ist, um als zweite Abbildungsvorschrift (138; 530; 850; 940), die einen Zusammenhang zwischen einem Druckwert oder einem Druckunterschied und einem Volumenwert oder einer Volumenänderung eines elastischen Fluidleiters oder Blutgefäßes beschreibt, eine sigmoidale Abbildungsvorschrift auszuwerten.

**8.** Druckmesser (100;700) gemäß einem der Ansprüche 6 bis 7, wobei der Druckwertbestimmer (120) ausgelegt ist, um die Amplitudeninformation (114) als Maß für eine Volumenänderung eines elastischen Fluidleiters oder eines Blutgefäßes zu verwenden, und
um ausgehend von dem ersten Druckwert oder Blutdruckwert (132) unter Berücksichtigung der durch die Amplitudeninformation beschriebenen Volumenänderung sowie unter Verwendung einer Abbildungsvorschrift, die einen nichtlinearen Zusammenhang zwischen Druck oder Blutdruck und einem Volumen eines elastischen Fluidleiters oder Blutgefäßes beschreibt, den zweiten Druckwert oder Blutdruckwert zu bestimmen.

**9.** Druckmesser (100;700) gemäß einem der Ansprüche 1 bis 8, wobei der Pulswellen-Charakterisierer (110) ausgelegt ist, um die Laufzeitinformation (112) so zu erhalten, dass die Laufzeitinformation eine Zeitdauer zwischen einem Punkt maximaler Steigung eines ersten Plethysmographen-Signals (710) und einem Punkt maximaler Steigung eines zweiten Plethysmographen-Signals (714) beschreibt, und um die Amplitudeninformation (114) so zu ermitteln, dass die Amplitudeninformation eine Amplitude (PA) von einem der Plethysmographen-Signale (710, 714) beschreibt;
wobei der Druckwertbestimmer (120) ausgelegt ist, um als den ersten Druckwert (132) einen systolischen Blutdruckwert basierend auf der Laufzeitinformation (112) und unter Verwendung der ersten Abbildung (134) zu erhalten; und
wobei der Druckwertbestimmer (120) ausgelegt ist, um als den zweiten Druckwert (136) einen diastolischen Blutdruckwert zu erhalten.

10. Druckmesser (100;700) gemäß einem der Ansprüche 1 bis 9, wobei der Druckmesser einen Kalibrator (750) aufweist;
wobei der Kalibrator ausgelegt ist, um eine Mehrzahl von Referenz-Laufzeitinformationen, die Laufzeiten von Pulswellen zwischen einem ersten Ort und einem zweiten Ort für eine Mehrzahl von Druckwerten beschreiben, und eine Mehrzahl von den Referenz-Laufzeitinformationen zugeordneten Referenz-Druckwerten zu empfangen,
um die erste Abbildung (134) unter Verwendung der Referenz-Laufzeitinformationen und der Referenz-Druckwerte festzulegen,
um einen qualitativen Verlauf der zweiten Abbildung (138) basierend auf der ersten Abbildung (134) festzulegen.

11. Druckmesser (100; 700) gemäß Anspruch 10, wobei der Kalibrator ausgelegt ist, um den qualitativen Verlauf der zweiten Abbildung durch Integration der ersten Abbildung bezüglich des Druckwertes zu bestimmen.

12. Druckmesser (100; 700) gemäß Anspruch 10 oder 11, wobei der Kalibrator ausgelegt ist, um eine Skalierung der zweiten Abbildung (138) basierend auf einer Information über eine Differenz zwischen zwei Druckwerten, die unterschiedliche Phasen des pulsierenden Flusses beschreiben, und basierend auf einer zugehörigen Amplitudeninformation, die eine Veränderung des Messsignals zwischen den zwei unterschiedlichen Phasen des pulsierenden Flusses beschreibt, festzulegen.

13. Druckmesser (100;700) gemäß einem der Ansprüche 1 bis 12, wobei der Druckmesser ausgelegt ist, um bei einer Kalibrierung basierend auf Referenz-Druckwerten, die einen systolischen Blutdruck beschreiben, und zugehörigen Referenz-Laufzeitwerten eine einen Gefäßabschnitt beschreibende Gauß-förmige Druck-Dehnbarkeits-Kurve zu bestimmen oder anzunähern,
um eine sigmoidale Druck-Volumen-Kurve durch Integration der Druck-Dehnbarkeits-Kurve zu erhalten, und
um unter Verwendung einer Differenz zwischen einem systolischen Referenz-Blutdruckwert und einem zugehörigen diastolischen Referenz-Blutdruckwert ein Verhältnis zwischen einer zu den Referenz-Blutdruckwerten gehörigen Amplitudeninformation und einer der Amplitudeninformation entsprechenden Volumendifferenz, wie sie durch die Druck-Volumen-Kurve beschrieben wird, als eine Skalierungsgröße zu bestimmen.

14. Druckmesser (100;700) gemäß Anspruch 13, wobei der Druckmesser ausgelegt ist,
um bei einer Blutdruckmessung eine auf einer Messung basierende Laufzeitinformation (PTT) zu quadrieren,
das Ergebnis des Quadrierens unter Verwendung der Druck-Dehnbarkeits-Kurve auf einen systolischen Blutdruckwert abzubilden, und
unter Verwendung des systolischen Blutdruckwerts, der Amplitudeninformation und der Druck-Volumen-Kurve einen diastolischen Blutdruckwert zu erhalten.

15. Verfahren (1000) zum Bestimmen von Druckwerten, die einen Druck eines in pulsierender Weise fließenden Fluids in zumindest zwei Phasen des pulsierenden Flusses beschreiben, unter Verwendung eines Druckmessers (100), mit folgenden Schritten:

Erhalten (1010) einer Laufzeitinformation, die eine Laufzeit einer Pulswelle zwischen einem ersten Ort und einem zweiten Ort beschreibt;
Erhalten (1020) einer Amplitudeninformation, die eine Veränderung eines auf der Pulswelle basierenden Mess-Signals zwischen zwei Phasen der Pulswelle beschreibt;
Bestimmen (1030) eines ersten Druckwerts, der einen Druck des Fluids in einer ersten Phase des pulsierenden Flusses beschreibt, basierend auf der Laufzeitinformation und unter Verwendung einer ersten Abbildung, die eine Laufzeit der Pulswelle auf den ersten Druckwert abbildet; und
Bestimmen (1040) eines zweiten Druckwerts, der einen Druck des Fluids in einer zweiten Phase des pulsierenden Flusses beschreibt, basierend auf dem ersten Druckwert und der Amplitudeninformation unter Verwendung einer zweiten Abbildung, die einen von dem ersten Druckwert abhängigen Zusammenhang zwischen dem ersten Druckwert, dem zweiten Druckwert und der Amplitudeninformation beschreibt.

16. Verfahren (1100) zum Kalibrieren eines Druckmessers, der ausgelegt ist, um zumindest einen Druckwert (122), der einen Druck eines in pulsierender Weise fließenden Fluids in einer Phase des pulsierenden Flusses beschreibt, unter Verwendung einer ersten Abbildung und einer zweiten Abbildung zu bestimmen, mit folgenden Schritten:

Bestimmen (1110) einer Mehrzahl von Referenz-Laufzeitinformationen, die Laufzeiten von Pulswellen zwischen einem ersten Ort und einem zweiten Ort für eine Mehrzahl von Druckwerten beschreiben, sowie einer Mehrzahl von zugehörigen Referenz-Druckwerten;
Bestimmen (1120) der ersten Abbildung, die einen Zusammenhang zwischen Laufzeiten der Pulswellen und zugehörigen Druckwerten beschreibt, unter Verwendung der Referenz-Laufzeitinformationen und der Referenz-Druckwerte; und

Bestimmen (1130) der zweiten Abbildung, die einen von einem ersten Druckwert abhängigen Zusammenhang zwischen dem ersten Druckwert, einem zweiten Druckwert und einer Amplitudeninformation beschreibt,

wobei ein qualitativer Verlauf der zweiten Abbildung basierend auf der ersten Abbildung festgelegt wird.

**17.** Computerprogramm zur Durchführung eines Verfahrens gemäß Anspruch 15 oder 16, wenn das Verfahren auf einem Computer ausgeführt wird.

**Claims**

**1.** A pressure gauge (100; 700) for determining at least one pressure value (122) describing a pressure of a fluid flowing in a pulsating manner in a phase of the pulsating flow, comprising:

a pulse wave characterizer (110) configured to obtain transmit time information (112) describing a transmit time of a pulse wave between a first location and a second location, and amplitude information (114) describing a change in a measurement signal, which is based on the pulse wave, between two phases of the pulse wave; and

a pressure value determiner (120) configured to obtain a first pressure value (132) describing a pressure of the fluid in a first phase of the pulsating flow on the basis of the transmit time information (112) and while using a first mapping (134) which maps the transmit time of the pulse wave to the first pressure value, and

to obtain a second pressure value (136) describing a pressure of the fluid in a second phase of the pulsating flow on the basis of the first pressure value (132) and the amplitude information (114) while using a second mapping (138),

the second mapping describing a relationship, which depends on the first pressure value (132), between the first pressure value (132), the second pressure value (136) and the amplitude information (114).

**2.** The pressure gauge (100; 700) as claimed in claim 1, the pressure gauge being a blood pressure gauge for determining a systolic blood pressure value or a diastolic blood pressure value;

wherein the pulse wave characterizer is configured to obtain the amplitude information such that the amplitude information describes the amplitude of a measurement signal based on the pulse wave;

wherein the pressure value determiner is a blood pressure value determiner that is configured to obtain, as the first pressure value, a first blood pressure value on the basis of the transmit time information and while using the first mapping, which maps the transmit time of the pulse wave to the first blood pressure value, and

that is configured to obtain, as the second pressure value, a second blood pressure value on the basis of the first blood pressure value and the amplitude information while using the second mapping;

wherein the second mapping describes a relationship, which depends on the first blood pressure value, between the amplitude information and a pressure amplitude of a pulse wave described by the measurement signal; and

wherein the second blood pressure value represents the systolic blood pressure value or the diastolic blood pressure value.

**3.** The pressure gauge (100; 700) as claimed in claim 1 or 2, wherein the pressure value determiner (120) is configured to evaluate, when the first pressure value (132) is determined, a first mapping specification (520; 810) which at least approximately describes a relationship between a blood pressure ($P_{tm}$) and a compliance (C) of a blood vessel.

**4.** The pressure gauge (100; 700) as claimed in claim 3, wherein the pressure value determiner (120) is configured to evaluate, as the first mapping specification (134; 520; 810) which at least approximately describes a relationship between a blood pressure ($P_{tm}$) and a compliance (C) of a blood vessel, a Gaussian-curve type mapping specification.

**5.** The pressure gauge (100; 700) as claimed in claim 3 or 4, wherein the pressure value determiner (120) is configured to square, when the first pressure value (132) is determined, a transmit time value (PTT) described by the transmit time information (122), so as to obtain the first blood pressure value (132) on the basis of the squared transmit time value while using the first mapping specification (134; 520; 810) describing the relationship between a blood pressure value ($P_{tm}$) and a compliance (C) of a blood vessel.

**6.** The pressure gauge (100; 700) as claimed in any of claims 1 to 5, wherein the pressure value determiner (120) is configured to evaluate, when the second pressure value (136) is determined, a second mapping specification (138; 530; 850; 940) describing a relationship between a pressure value or a pressure difference and a volume value or a change in the volume of an elastic fluid conductor or a blood vessel.

**7.** The pressure gauge (100; 700) as claimed in claim 6, wherein the pressure value determiner (120) is configured to evaluate, as the second mapping specification (138; 530; 850; 940) describing a relationship between a pressure value or a pressure difference and a volume value or a change in the volume of an elastic fluid conductor or a blood vessel, a sigmoidal mapping specification.

8. The pressure gauge (100; 700) as claimed in one of claims 6 to 7, wherein the pressure value determiner (120) is configured to use the amplitude information (114) as a measure of a change in the volume of an elastic fluid conductor or of a blood vessel, and to determine the second pressure value or blood pressure value while starting from the first pressure value or blood pressure value (132) and while taking into account the change in volume described by the amplitude information and while using a mapping specification describing a non-linear relationship between pressure or blood pressure and a volume of an elastic fluid conductor or blood vessel.

9. The pressure gauge (100; 700) as claimed in any of claims 1 to 8, wherein the pulse wave characterizer (110) is configured to obtain the transmit time information (112) such that the transmit time information describes a duration between a point of a maximum inclination of a first plethysmograph signal (710) and a point of a maximum inclination of a second plethysmograph signal (714), and to determine the amplitude information (114) such that the amplitude information describes an amplitude (PA) of one of the plethysmograph signals (710, 714);
wherein the pressure value determiner (120) is configured to obtain, as the first pressure value (132), a systolic blood pressure value on the basis of the transmit time information (112) and while using the first mapping (134); and
wherein the pressure value determiner (120) is configured to obtain, as the second pressure value (136), a diastolic blood pressure value.

10. The pressure gauge (100; 700) as claimed in any of claims 1 to 9, wherein the pressure gauge comprises a calibrator (750);
wherein the calibrator is configured to receive a plurality of pieces of reference transmit time information which describe transmit times of pulse waves between a first location and a second location for a plurality of pressure values, and a plurality of reference pressure values associated with the reference transmit time information,
to specify the first mapping (134) while using the reference transmit time information and the reference pressure values,
to specify a qualitative course of the second mapping (138) on the basis of the first mapping (134).

11. The pressure gauge (100; 700) as claimed in claim 10, wherein the calibrator is configured to determine the qualitative course of the second mapping by integrating the first mapping with regard to the pressure value.

12. The pressure gauge (100; 700) as claimed in claim 10 or 11, wherein the calibrator is configured to specify a scaling of the second mapping (138) on the basis of information about a difference between two pressure values which describe different phases of the pulsating flow, and on the basis of associated amplitude information describing a change in the measurement signal between the two different phases of the pulsating flow.

13. The pressure gauge (100; 700) as claimed in any of claims 1 to 12, the pressure gauge being configured to determine or approximate, during a calibration, on the basis of reference pressure values describing a systolic blood pressure and on the basis of associated reference transmit time values, a Gaussian pressure/compliance curve which describes a vascular section,
to obtain a sigmoidal pressure/volume curve by integrating the pressure/compliance curve, and
to determine a ratio between amplitude information belonging to the reference blood pressure values and a volume difference as is described by the pressure/volume curve and which corresponds to the amplitude information, as a scaling quantity while using a difference between a systolic reference blood pressure value and an associated diastolic reference blood pressure value.

14. The pressure gauge (100; 700) as claimed in claim 13, the pressure gauge being adapted
to square, during blood pressure measurement, transmit time information (PTT) that is based on a measurement,
to map the result of the squaring to a systolic blood pressure value while using the pressure/compliance curve, and
to obtain a diastolic blood pressure value while using the systolic blood pressure value, the amplitude information and the pressure/volume curve.

15. A method (1000) of determining, while using a pressure gauge (100), pressure values which describe a pressure of a fluid that is flowing in a pulsating manner in at least two phases of the pulsating flow, comprising the following steps:

obtaining (1010) transmit time information describing a transmit time of a pulse wave between a first location and a second location;
obtaining (1020) amplitude information describing a change in a measurement signal, based on a pulse wave, between two phases of the pulse wave;

determining (1030) a first pressure value, which describes a pressure of the fluid in a first phase of the pulsating fluid, on the basis of the transmit time information and while using a first mapping, which maps a transmit time of the pulse wave to the first pressure value; and

determining (1040) a second pressure value, which describes a pressure of the fluid in a second phase of the pulsating flow, on the basis of the first pressure value and of the amplitude information, while using a second mapping, which describes a relationship - which is dependent on the first pressure value - between the first pressure value, the second pressure value and the amplitude information.

16. A method (1100) of calibrating a pressure gauge configured to determine, while using a first mapping and a second mapping, at least one pressure value (122) which describes a pressure of a fluid flowing in a pulsating manner in a phase of the pulsating flow, comprising the following steps:

determining (1110) a plurality of pieces of reference transmit time information describing transmit times of pulse waves between a first location and a second location for a plurality of pressure values, and a plurality of associated reference pressure values;

determining (1120) the first mapping, which describes a relationship between transmit times of the pulse waves and associated pressure values, while using the reference transmit time information and the reference pressure values; and

determining (1130) the second mapping, which describes a relationship, that is dependent on a first pressure value, between the first pressure value, a second pressure value and amplitude information,

a qualitative course of the second mapping being specified on the basis of the first mapping.

17. A computer program for performing a method as claimed in claim 15 or 16, when the method is performed on a computer.

## Revendications

1. Manomètre (100; 700) pour déterminer au moins une valeur de pression (122) qui décrit une pression d'un fluide circulant de manière pulsée dans une phase du flux pulsé, aux caractéristiques suivantes:

un caractériseur d'ondes de pulsation (110) qui est conçu pour obtenir une information de durée (112) qui décrit une durée d'une onde d'impulsion entre un premier endroit et un deuxième endroit, et une information d'amplitude (114) qui décrit une variation d'un signal de mesure basé sur l'onde de pulsation entre deux phases de l'onde de pulsation; et

un déterminateur de valeur de pression (120) qui est conçu pour obtenir une première valeur de pression (132) décrivant une pression du fluide dans une première phase de flux pulsé, sur base de l'information de durée (112) et à l'aide d'une première reproduction (134) qui reproduit la durée de l'onde de pulsation sur la première valeur de pression, et

pour obtenir une deuxième valeur de pression (136) décrivant une pression du fluide dans une deuxième phase du flux pulsé, sur base de la première valeur de pression (132) et de l'information d'amplitude (114), à l'aide d'une deuxième reproduction (138),

dans lequel la deuxième reproduction décrit un rapport, en fonction de la première valeur de pression (132), entre la première valeur de pression (132), la deuxième valeur de pression (136) et l'information d'amplitude (114).

2. Manomètre (100; 700) selon la revendication 1, dans lequel le manomètre est un sphygmomanomètre destiné à déterminer une valeur de pression sanguine systolique ou une valeur de pression sanguine diastolique;

dans lequel le caractériseur d'ondes d'impulsion est réalisé pour obtenir l'information d'amplitude de sorte que l'information d'amplitude décrive l'amplitude d'un signal de mesure basé sur l'onde d'impulsion;

dans lequel le déterminateur de valeur de pression est un déterminateur de valeur de pression sanguine,

qui est conçu pour obtenir, comme première valeur de pression, une première valeur de pression sanguine sur base de l'information de durée et à l'aide de la première représentation qui reproduit la durée de l'onde d'impulsion sur la première valeur de pression sanguine, et

qui est conçu pour obtenir, comme deuxième valeur de pression, une deuxième valeur de pression sanguine sur base de la première valeur sanguine et de l'information d'amplitude, à l'aide de la deuxième reproduction;

dans lequel la deuxième reproduction décrit un rapport, en fonction de la première valeur de pression sanguine, entre l'information de l'amplitude et une amplitude de pression d'une onde de pression décrite par le signal de mesure; et

dans lequel la deuxième valeur de pression sanguine représente la valeur de pression sanguine systolique ou la valeur de pression sanguine diastolique.

**3.** Manomètre (100; 700) selon la revendication 1 ou 2, dans lequel le déterminateur de valeur de pression (120) est conçu pour évaluer, lors de la détermination de la première valeur de pression (132), une première règle de reproduction (520; 810) qui décrit de manière au moins approximative un rapport entre une pression sanguine ($P_{tm}$) et une aptitude à la dilatation (C) d'un vaisseau sanguin.

**4.** Manomètre (100; 700) selon la revendication 3, dans lequel le déterminateur de valeur de pression (120) est conçu pour évaluer du moins environ, comme première règle de reproduction (134; 520; 810) décrivant un rapport entre une pression sanguine ($P_{tm}$) et une aptitude à la dilatation (C) d'un vaisseau sanguin, une règle de reproduction en forme du courbe gaussienne.

**5.** Manomètre (100; 700) selon la revendication 3 ou 4, dans lequel le déterminateur de valeur de pression (120) est conçu pour élever au carré, lors de la détermination de la première valeur de pression (132), une valeur de durée (PTT) décrite par l'information de durée (122), et pour obtenir sur base de la valeur de durée élevée au carré, à l'aide de la première règle de reproduction (134; 520; 810) décrivant le rapport entre une valeur de pression sanguine ($P_{tm}$) et une aptitude à la dilatation (C) d'un vaisseau sanguin, la première valeur de pression sanguine (132).

**6.** Manomètre (100; 700) selon l'une des revendications 1 à 5, dans lequel le déterminateur de valeur de pression (120) est conçu pour évaluer, lors de de la détermination de la deuxième valeur de pression (136), une deuxième règle de reproduction (138; 530; 850; 940) décrivant un rapport entre une valeur de pression ou une différence de pression et une valeur de volume ou une variation de volume d'un conducteur de fluide élastique ou d'un vaisseau sanguin.

**7.** Manomètre (100; 700) selon la revendication 6, dans lequel le déterminateur de valeur de pression (120) est conçu pour évaluer, comme deuxième règle de reproduction (138; 530; 850; 940) décrivant un rapport entre une valeur de pression ou une différence de pression et une valeur de volume ou une variation de volume d'un conducteur de fluide élastique ou d'un vaisseau sanguin, une règle de reproduction sigmoïdale.

**8.** Manomètre (100; 700) selon l'une des revendications 6 à 7, dans lequel le déterminateur de valeur de pression (120) est conçu pour utiliser l'information d'amplitude (114) comme mesure pour une variation de volume d'un conducteur de fluide élastique ou d'un vaisseau sanguin, et pour déterminer, partant de la première valeur de pression ou valeur de pression sanguine (132), en tenant compte de la variation de volume décrite par l'information d'amplitude ainsi qu'à l'aide d'une règle de reproduction décrivant un rapport non linéaire entre la pression ou la pression sanguine et un volume d'un conducteur de fluide élastique ou d'un vaisseau sanguin, la deuxième valeur de pression ou valeur de pression sanguine.

**9.** Manomètre (100; 700) selon l'une des revendications 1 à 8, dans lequel le caractériseur d'ondes d'impulsion (110) est conçu pour obtenir l'information de durée (112) de sorte que l'information de durée décrive une durée entre un point de montée maximale d'un premier signal de pléthysmographe (710) et un point de montée maximale d'un deuxième signal de pléthysmographe (714), et pour déterminer l'information d'amplitude (114) de sorte que l'information d'amplitude décrive une amplitude (PA) de l'un des signaux de pléthysmographe (710, 714); dans lequel le déterminateur de valeur de pression (120) est conçu pour obtenir, comme première valeur de pression (132), une valeur de pression sanguine systolique sur base de l'information de durée (112) et à l'aide de la première reproduction (134); et dans lequel le déterminateur de valeur de pression (120) est conçu pour obtenir, comme deuxième valeur de pression (136), une valeur de pression diastolique.

**10.** Manomètre (100; 700) selon l'une des revendications 1 à 9, dans lequel le manomètre présente un étalonneur (750); dans lequel l'étalonneur est conçu pour recevoir une pluralité d'informations de durée de référence décrivant les durées d'ondes d'impulsion entre un premier endroit et un deuxième endroit pour une pluralité de valeurs de pression, et une pluralité de valeurs de pression de référence associées aux informations de durée de référence, et pour fixer la première reproduction (134) à l'aide des informations de durée de référence et des valeurs de pression de référence, pour fixer une évolution qualitative de la deuxième reproduction (138) sur base de la première reproduction (134).

**11.** Manomètre (100; 700) selon la revendication 10, dans lequel l'étalonneur est conçu pour déterminer l'évolution qualitative de la deuxième reproduction par intégration de la première reproduction concernant la valeur de pression.

**12.** Manomètre (100; 700) selon la revendication 10 ou 11, dans lequel l'étalonneur est conçu pour fixer un échelonnement de la deuxième reproduction (138) sur base d'une information sur une différence entre deux valeurs de pression décrivant des phases différentes du flux pulsé, et sur base d'une information d'amplitude associée décrivant une variation du signal de mesure entre les deux phases différentes du flux pulsé.

13. Manomètre (100; 700) selon l'une des revendications 1 à 12, dans lequel le manomètre est conçu pour déterminer ou approcher, lors d'un étalonnage sur base de valeurs de pression de référence décrivant une pression sanguine systolique et de valeurs de durée de référence associées, une courbe de pression-aptitude à la dilatation de forme gaussienne décrivant un segment de vaisseau,
pour obtenir une courbe de pression-volume sigmoïdale par intégration de la courbe de pression-aptitude à la dilatation, et
pour obtenir, à l'aide d'une différence entre une valeur de pression sanguine de référence systolique et une valeur de pression sanguine de référence diastolique associée, un rapport entre une information d'amplitude associée aux valeurs de pression sanguine de référence et une différence de volume correspondant à l'information d'amplitude telle que décrite par la courbe pression-volume, comme grandeur d'échelonnement.

14. Manomètre (100; 700) selon la revendication 13, dans lequel le manomètre est conçu
pour élever au carré, lors d'une mesure de pression sanguine, une information de durée basée sur une mesure (PTT),
reproduire le résultat de l'élévation au carré à l'aide de la courbe de pression-aptitude à la dilatation sur une valeur de pression sanguine systolique, et
obtenir, à l'aide de la valeur de pression sanguine systolique, de l'information d'amplitude et de la courbe de pression-volume, une valeur de pression sanguine diastolique.

15. Procédé (1000) pour déterminer des valeurs de pression décrivant une pression d'un fluide circulant de manière pulsée dans au moins deux phases du flux pulsé, à l'aide d'un manomètre (100), aux étapes suivantes consistant à:

    obtenir (1010) une information de durée décrivant une durée d'une onde d'impulsion entre un premier endroit et un deuxième endroit;
    obtenir (1020) une information d'amplitude décrivant une variation d'un signal de mesure basé sur l'onde d'impulsion entre deux phases de l'onde d'impulsion;
    déterminer (1030) une première valeur de pression décrivant une pression du fluide dans une première phase du flux pulsé, sur base de l'information de durée et à l'aide d'une première reproduction reproduisant une durée de l'onde d'impulsion sur la première valeur de pression;

et
déterminer (1040) une deuxième valeur de pression décrivant une pression du fluide dans une deuxième phase du flux pulsé, sur base de la première valeur de pression et de l'information d'amplitude à l'aide d'une deuxième reproduction décrivant un rapport, en fonction de la première valeur de pression, entre la première valeur de pression, la deuxième valeur de pression et l'information d'amplitude.

16. Procédé (1100) d'étalonnage d'un manomètre qui est conçu pour déterminer au moins une valeur de pression (122) décrivant une pression d'un fluide circulant de manière pulsée dans une phase du flux pulsé, à l'aide d'une première reproduction et d'une deuxième reproduction, aux étapes suivantes consistant à:

    déterminer (1110) une pluralité d'informations de durée de référence décrivant des durées d'ondes d'impulsion entre un premier endroit et un deuxième endroit pour une pluralité de valeurs de pression, ainsi qu'une pluralité de valeurs de pression de référence associées;
    déterminer (1120) la première reproduction décrivant un rapport entre les durées des ondes d'impulsion et les valeurs de pression associées, à l'aide des informations de durée de référence et des valeurs de pression de référence; et
    déterminer (1130) la deuxième reproduction décrivant un rapport, en fonction d'une première valeur de pression, entre la première valeur de pression, une deuxième valeur de pression et une information d'amplitude,
    dans lequel une évolution qualitative de la deuxième reproduction est fixée sur base de la première reproduction.

17. Programme d'ordinateur pour réaliser un procédé selon la revendication 15 ou 16 lorsque le procédé est exécuté sur un ordinateur.

FIGUR 1

EP 2 240 072 B1

FIGUR 2A

FIGUR 2B

300

PG1
310

PG2

330   320

340

# FIGUR 3

PG1
414
410   400

PG1max
418
416
PA
PG1min

t
412

PG2
424
420
426
428

T₁   PTT   T₂

t
422

# FIGUR 4

# FIGUR 5

## FIGUR 6A

V oder MS

600

V(PTT') + ΔV oder MS(PTT') + PA

ΔV oder PA

V(PTT') oder MS(PTT')

534

530

$P_{tm}(PTT')$

532

$P_{tm}$

$P_{tm2}(PTT', PA)$

## FIGUR 6B

V oder MS

650

V(PTT'') oder MS(PTT'') — (1-x1)·PA

(x1)·PA

z.B.: $x_1$ = 50%

530

$P_{tm}(PTT'')$

$P_{tm}$

$P_{tm2}(PTT'', PA)$   $P_{tm3}(PTT'', PA)$

FIGUR 7

(Referenzdruckwert; Referenzlaufzeitwert)

$P_{tm,refi}$ ↓ $PTT_{refi}$

x Erste Abbildung festgelegt

x Qualitativer Verlauf der 2.Abbildung entspricht dem Integral
des Verlaufs der 1.Abbildung

Achsenskalierung
bzw. Skalierungsgröße noch
unbekannt

Kurvenverlauf basierend
auf 1.Abbildung
(ohne weitere Information ableitbar)

Achsenskalierung basierend
auf 1.Abbildung
(ohne weitere Information ableitbar)

**FIGUR 8A**

FIGUR 8B

FIGUR 8C

FIGUR 9

1000

Erhalten einer Laufzeitinformation, die eine Laufzeit einer Pulswelle zwischen einem 1.Ort und einem 2.Ort beschreibt — 1010

Erhalten einer Amplitudeninformation, die eine Veränderung eines auf der Pulswelle basierenden Meßsignals zwischen zwei Phasen der Pulswelle beschreibt — 1020

Bestimmen eines ersten Druckwertes, der einen Druck des Fluids in einer ersten Phase des pulsierenden Flusses beschreibt, basierend auf der Laufzeitinformation und unter Verwendung einer Abbildung, die die Laufzeit der Pulswelle auf den 1. Druckwert abbildet — 1030

Bestimmen eines zweiten Druckwertes, der einen Druck des Fluids in einer zweiten Phase des pulsierenden Flusses beschreibt, basierend auf dem 1. Druckwert und der Amplitudeninformation und unter Verwendung einer Abbildung, die einen von dem 1.Druckwert in nichtlinearer Weise abhängigen Zusammenhang zwischen dem 1. Druckwert, dem 2. Druckwert und der Amplituden- informationbeschreibt. — 1040

# FIGUR 10

Bestimmen einer Mehrzahl von Referenz-Laufzeitinformationen, die Laufzeiten von Pulswellen zwischen einem ersten Ort und einem zweiten Ort für eine für eine Mehrzahl von Druckwerten beschreiben, sowie eine Mehrzahl von zugehörigen Referenz-Druckwerten

~1110

Bestimmen einer ersten Abbildung, die einen Zusammenhang zwischen Laufzeiten der Pulswellen und zugehörigen Druckwerten beschreibt, unter Verwendung der Referenz-Laufzeitinformation und der Referenz-Druckwerte

~1120

Bestimmen einer zweiten Abbildung, die eine Abbildung eines Meßsignals, das zumindest eine Pulswelle beschreibt, auf einen Druckwert ermöglicht, basierend auf der ersten Abbildung, so dass ein qualitativer Verlauf der zweiten Abbildung einen druckabhängigen Zusammenhang zwischen lokalen Volumenwerten oder lokalen Volumenänderungen eines Fluidleiters, in dem sich die Pulswelle ausbreitet, und zugehörigen Druckwerten oder Druckunterschieden beschreibt, wobei eine Skalierungsgröße der zweiten Abbildung basierend auf zumindest zwei Referenzdruckwerten und zumindest zwei entsprechenden Signalwerten eines Meßsignals festgelegt wird.

~1130

# FIGUR 11

**EP 2 240 072 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6736789 B1
- GB 2394178 A
- US 7029447 B2
- US 6648828 B2
- US 6331162 B1
- KR 000100697211B A
- KR 102006069032A A
- US 4245648 A
- DE 10061189 A1
- US 20050261593 A1
- EP 1157658 A1
- US 20040162493 A1
- EP 2030564 A2